(19) Europäisches Patentamt
European Patent Office
Office européen des brevets

(11) Veröffentlichungsnummer: **0 035 744**
**A1**

(12) # EUROPÄISCHE PATENTANMELDUNG

(21) Anmeldenummer: **81101546.0**

(22) Anmeldetag: **04.03.81**

(51) Int. Cl.$^3$: **C 07 D 307/91**
A 01 N 43/12
//C07C69/025

(30) Priorität: **12.03.80 DE 3009376**

(43) Veröffentlichungstag der Anmeldung:
**16.09.81 Patentblatt 81/37**

(84) Benannte Vertragsstaaten:
**AT BE CH DE FR GB IT LI NL SE**

(71) Anmelder: **BASF Aktiengesellschaft**
**Carl-Bosch-Strasse 38**
**D-6700 Ludwigshafen(DE)**

(72) Erfinder: **Rentzea, Costin, Dr.**
**Neuenheimer Landstrasse 72**
**D-6900 Heidelberg(DE)**

(72) Erfinder: **Ammermann, Eberhard, Dr.**
**Sachsenstrasse 3**
**D-6700 Ludwigshafen(DE)**

(72) Erfinder: **Feuerherd, Karl-Heinz, Dr.**
**Berner Weg 34**
**D-6700 Ludwigshafen(DE)**

(72) Erfinder: **Sauter, Hubert, Dr.**
**Neckarpromenade**
**D-6800 Mannheim 1(DE)**

(72) Erfinder: **Wuerzer, Bruno, Dr. Dipl.-Landwirt**
**Ruedigerstrasse 13**
**D-6701 Otterstadt(DE)**

(54) **Dibenzofuranoxyalkancarbonsäureester, Verfahren zu ihrer Herstellung, diese enthaltende Herbizide und ihre Anwendung als Herbizide.**

(57) Die vorliegende Anmeldung betrifft Dibenzofuran-3-oxyalkancarbonsäure-Derivate der allgemeinen Formel

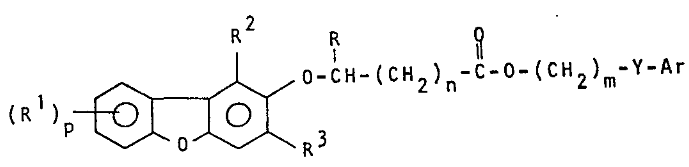

in welcher
- $R^1$ Halogen, eine gegebenenfalls substituierte Alkyl- oder Alkoxygruppe, Cyan oder Nitro bedeutet,
- $R^2$ und $R^3$ Wasserstoff, Halogen oder Nitro bedeuten,
- R Wasserstoff, Methyl oder Ethyl,
- m die Zahlen 2, 3, 4, 5 oder 6
- n die Zahlen 0 oder 2
- p die Zahlen 1 oder 2
- p die Zahlen 1 oder 2 und
- Y ein Sauerstoff- oder Schwefelatom bedeuten und
- Ar einen heterocyclischen Rest oder einen Arylrest bedeutet, der gegebenenfalls substituiert sein kann, Verfahren zu ihrer Herstellung und ihre Anwendung als Herbizide.

EP 0 035 744 A1

Dibenzofuranoxyalkancarbonsäureester, Verfahren zu ihrer Herstellung, diese enthaltende Herbizide und ihre Anwendung als Herbizide

Die vorliegende Erfindung betrifft wertvolle neue Dibenzofuran-3-oxyalkancarbonsäure-Derivate, Verfahren zu ihrer Herstellung, Herbizide, welche diese Verbindungen als Wirkstoffe enthalten, sowie Verfahren zur Bekämpfung unerwünschten Pflanzenwuchses mit diesen Verbindungen.

Die Entdeckung der herbiziden Wirkung von Phenoxycarbonsäuren im Jahre 1942 ermöglichte die Bekämpfung von zahlreichen breitblättrigen Unkräutern in Kulturen von Nutzpflanzen aus der Familie der Gramineen wie Getreide, Mais oder Reis. Phenoxycarbonsäure-Derivate beispielsweise das Dimethylammoniumsalz der 2-Methyl-4-chlorphenoxy-$\alpha$-propionsäure (DE-AS 10 64 286) ist ein wichtiger bekannter herbizider Wirkstoff.

Trotz der weltweiten Anwendung der Phenoxycarbonsäuren darf nicht verkannt werden, daß ihre Anwendung nicht ohne Risiko für die Kulturpflanzen erfolgt. Man versucht, dieses Risiko durch möglichst präzise Einhaltung der Aufwandmengen bei der Ausbringung der Wirkstoffe bei bestimmten wenig empfindlichen Wachstumsstadien der Kulturpflanzen herabzusetzen.

Erst 1979 wurde es bekannt, Dibenzofuranyloxyalkancarbonsäure-Derivate als Herbizide zu verwenden (DE-OS 28 23 424).

Es wurde nun gefunden, daß neue Dibenzofuranoxyalkancarbonsäureester der allgemeinen Formel

Sws/BL

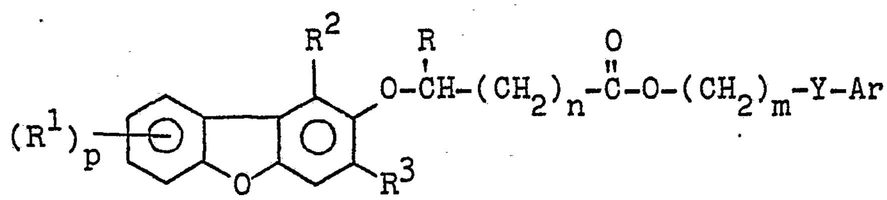

in welcher

$R^1$ Halogen (z.B. Fluor, Chlor, Brom oder Jod), eine gegebenenfalls halogensubstituierte Alkyl- oder Alkoxygruppe mit 1 bis 4 C-Atomen (z.B. Methyl, Ethyl, tert.-Butyl, Methoxy, Ethoxy, Trifluormethyl, Tetrafluorethoxy), Cyan oder Nitro bedeutet,

$R^2$ und $R^3$ gleich oder verschieden sind und unabhängig voneinander Wasserstoff, Halogen (z.B. Fluor, Chlor oder Brom) oder Nitro bedeuten,

R Wasserstoff, Methyl oder Ethyl,

p die Zahlen 1 oder 2,

n die Zahlen 0 oder 2,

m die Zahlen 2, 3, 4, 5 oder 6 und

Y ein Sauerstoff- oder Schwefelatom bedeuten und

Ar 3-Dibenzofuranyl, 1,2- oder 1,4-Biphenyl, 1- oder 2-Naphthyl oder Phenyl bedeuten, wobei der Phenylrest durch Halogen (z.B. Fluor, Chlor, Brom oder Jod), durch gegebenenfalls halogensubstituierte Alkyl-, Alkenyl-, Alkoxy- oder Alkylthiogruppen mit 1 bis 5 C-Atomen (z.B. Methyl, Ethyl, Propyl, tert.-Butyl, Allyl, 2-Butenyl, Methoxy, Ethoxy, Butoxy, Methylthio, Trifluormethyl, Tetrafluorethoxy) oder durch Acetyl, Cyan oder Nitro substituiert sein kann,

bei guter herbizider Wirksamkeit ein besonders hohes Maß an Verträglichkeit für landwirtschaftliche Kulturpflanzen aus der Familie der Gramineen aufweisen.

Es wurde weiterhin gefunden, daß man die neuen Verbindungen erhält,

1. wenn man einen Ester der Formel

$$Z-\overset{R}{\overset{\|}{C}}H-(CH_2)_n\overset{O}{\overset{\|}{C}}-O-(CH_2)_m-Y-Ar$$

in welcher
Z ein Halogenatom wie Chlor, Brom oder Jod und
R, Y, Ar, m und n die oben angegebenen Bedeutungen haben,

a) mit einem substituierten Dibenzofuranol der Formel

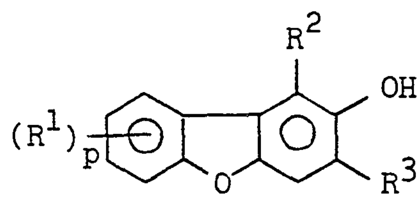

oder

b) mit dem Metallsalz eines substituierten Dibenzofuranols

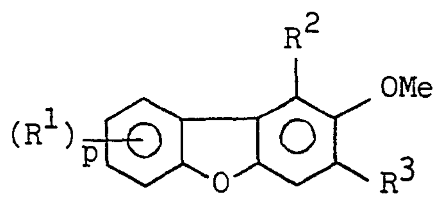

in welcher
Me Lithium, Natrium oder Kalium bedeutet und
$R^1$, $R^2$, $R^3$ und p die oben angegebenen Bedeutungen haben, gegebenenfalls in Gegenwart eines Lösungs- oder Verdünnungsmittels, gegebenenfalls unter Zusatz einer anorganischen Base und gegebenenfalls unter Zusatz eines Reaktionsbeschleunigers, bei Temperaturen zwischen 20 und 150°C umsetzt. Zu den bevorzugten Lösungs- und Verdün-

nungsmitteln gehören Halogenkohlenwasserstoffe, beispielsweise Methylenchlorid, Chlorbenzol, Chlornaphthalin; aliphatische oder aromatische Kohlenwasserstoffe wie Petrolether, Cyclohexan, Benzol, Toluol, Xylol oder Cumol; Ester wie Essigsäureethylester; Nitrile wie Acetonitril; Sulfoxide wie Dimethylsulfoxid; Ketone wie Aceton, Methylethylketon oder Cyclopentanon; Ether wie Diethylether, Tetrahydrofuran oder Dioxan oder entsprechende Gemische.

Geeignete anorganische Basen, die gegebenenfalls auch als säurebindende Mittel bei der Reaktion verwendet werden können, sind beispielsweise Alkalihydroxide wie Lithium-, Natrium- oder Kaliumhydroxid; Alkalicarbonate wie Natrium- oder Kaliumcarbonat oder Natrium- und Kaliumhydrogencarbonat oder Alkalihydride wie Natriumhydrid. Es können aber auch andere übliche Basen verwendet werden.

Als Reaktionsbeschleuniger kommen vorzugsweise Metallhalogenide wie Natriumbromid, Natriumjodid oder Kaliumjodid, quaternäre Ammoniumsalze wie Tetrabutylammoniumchlorid, -bromid oder -jodid, Benzyl-triethylammonium-chlorid oder -bromid oder Methyltrioctylammonium-chlorid oder -bromid oder Kronenether wie 12-Krone-4, 15-Krone-5, 18-Krone-6, Dibenzo-18-krone-6 oder Dicyclohexano-18-krone-6 in Frage.

Die Umsetzung wird im allgemeinen bei Temperaturen zwischen 20 und 150°C, drucklos oder unter Druck, kontinuierlich oder diskontinuierlich durchgeführt.

Die oben genannten Ester sind neu. Sie können nach bekannten Verfahren herstellt werden entweder

i) durch Veresterung von α-Halogen-fettsäuren mit Alkoholen der Formel

$$Z-\overset{R}{\overset{|}{C}}H-(CH_2)_n-COOH + HO-(CH_2)_m-Y-Ar$$

worin Z, R, Y, Ar, m und n die oben angegebenen Bedeutungen haben, beispielsweise nach Newman u.a., J. Amer. Chem. Soc., 77, 946 (1955), oder

ii) durch Umsetzung von Säurehalogeniden der Formel

$$Z-\overset{R}{\overset{|}{C}}H-(CH_2)_n-COZ$$

mit den oben genannten Alkoholen, worin Z, R und n die oben angegebenen Bedeutungen haben, beispielsweise nach Newman u.a. (loc. cit.), oder

iii) durch Umsetzung von Estern der Formel

$$Z-\overset{R}{\overset{|}{C}}H-(CH_2)_n-COOCH_3$$

mit den oben genannten Alkoholen, worin Z, R und n die oben angegebenen Bedeutungen haben, beispielsweise nach Rinehart, Organic Synthesis, Coll. Vol. IV, S. 122 (1963).

Dibenzofuranole sind bekannt und z.B. von Schimmelschmidt in Liebigs Annalen 566, 184 ff (1950) beschrieben. Sie lassen sich nach den dort beschriebenen Verfahren bzw. nach entsprechend abgewandelten Methoden herstellen.

Es wurde weiterhin gefunden, daß man die neuen Verbindungen erhält

2. wenn man einen Alkohol der Formel

$$HO-(CH_2)_m-Y-Ar$$

in welcher
Ar, Y und m die oben angegebenen Bedeutungen haben

a) mit einer Dibenzofuranoxyfettsäure der Formel

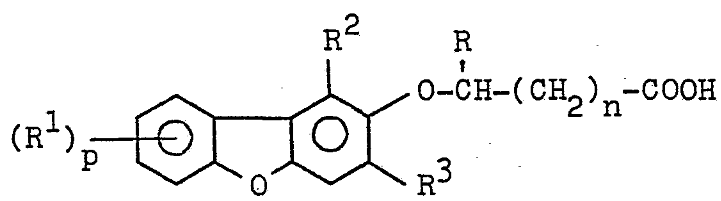

in welcher
$R^1$, $R^2$, $R^3$, R, n und p die oben angegebenen Bedeutungen haben, gegebenenfalls in Gegenwart eines Lösungs- oder Verdünnungsmittels, gegebenenfalls unter Zusatz einer anorganischen, organischen oder polymeren starken Säure und gegebenenfalls unter Wasserausscheidung, bei Temperaturen zwischen 20 und 150°C verestert, oder

b) mit einem Dibenzofuranoxyfettsäure-halogenid der Formel

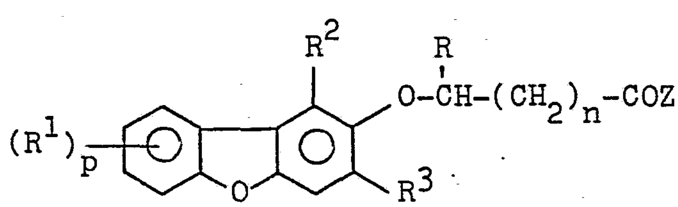

in welcher

$R^1$, $R^2$, $R^3$, R, Z, n und p die oben angegebenen Bedeutungen haben, gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers, bei Temperaturen zwischen 0 und 120°C umsetzt, oder

c) mit einem Dibenzofuranoxyfettsäureester der Formel

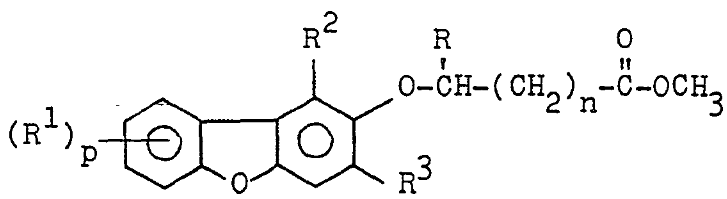

in welcher $R^1$, $R^2$, $R^3$, R, Z, n und p die oben angegebenen Bedeutungen haben, gegebenenfalls in Gegenwart eines Lösungs- oder Verdünnungsmittels, gegebenenfalls unter Zusatz einer starken anorganischen oder organischen Base oder einer starken anorganischen oder organischen Säure oder einer Lewis-Säure oder einem Metalloxid oder -alkoxid, bei Temperaturen von 0 bis 150°C umestert.

Die oben genannten Alkohole sind bekannt (s. beispielsweise N.K. Bliznyuk u.a. J. Gen. Chem. UdSSR 36, 480 (1966)). Sie lassen sich nach den dort beschriebenen Verfahren herstellen. Die anderen Verbindungen sind aus der DE-OS 27 32 924 und DE-OS 28 23 424 bekannt.

Als bevorzugte Lösungs- und Verdünnungsmittel für das Verfahren 2a werden z.B. die Alkohole in zwei- bis zehnfach molarem Überschuß oder aliphatische oder aromatische Kohlen-

wasserstoffe wie Hexan, Heptan, Octan, Cyclohexan, Methylcyclohexan, Benzol, Toluol, Xylol; Halogenkohlenwasserstoffe wie Chlorbenzol oder Dichlorbenzole oder entsprechende Gemische verwendet.

Als saure Katalysatoren eignen sich Mineralsäuren wie Chlorwasserstoff, Bromwasserstoff, Schwefelsäure, Phosphorsäure oder organische Säure wie p-Toluolsulfonsäure oder Trifluormethylsulfonsäure oder saure Ionenaustauscher.

Als bevorzugte, gegenüber den Reaktionsteilnehmern inerte Lösungs- und Verdünnungsmittel für das Verfahren 2b werden beispielsweise aliphatische oder aromatische Kohlenwasserstoffe wie Pentan, Hexan, Cyclohexan, Petrolether, Benzol, Toluol oder Xylole; Halogenkohlenwasserstoffe wie Methylenchlorid, Chloroform, 1,2-Dichlorethan, Chlorbenzol; Ketone wie Aceton oder Methylethylketon; Ether wie Diethylether, Dimethoxyethan, Tetrahydrofuran oder Dioxan; Ester wie Essigsäureethylester; Nitrile wie Acetonitril oder Sulfoxide wie Dimethylsulfoxid verwendet.

Geeignete anorganische oder organische Basen, die gegebenenfalls auch als Säure-bindende Mittel bei der Reaktion verwendet werden können, sind beispielsweise Alkali- und Erdalkalicarbonate wie Natrium- oder Kaliumbicarbonat, Natrium oder Kaliumcarbonat, Calciumcarbonat, Bariumcarbonat; Borate wie Natriumborat; Phosphate wie Natrium- oder Kalium-di- oder triphosphat oder Amine wie Triethylamin, N,N-Dimethylanilin, N,N-Dimethylcyclohexylamin, N-Methylpiperidin oder Pyridin. Es können aber auch andere übliche Basen verwendet werden.

Als Reaktionsbeschleuniger kommen vorzugsweise Metallhalogenide wie Natriumbromid oder Kaliumjodid, Azole wie Imidazol oder 1,2,4-Triazol oder Pyridine wie 4-Dimethylaminopyridin in Frage.

Als bevorzugte Lösungs- und Verdünnungsmittel für das Verfahren 2c werden z.B. die Alkohole in zwei- bis zehnfach molarem Überschuß oder aliphatische oder aromatische Kohlenwasserstoffe wie Hexan, Octan, Cyclohexan, Benzol, Toluol, Xylole, Cumol oder entsprechende Gemische verwendet.

Als Umesterungskatalysatoren für die Umsetzung 2c eignen sich anorganische oder organische Basen, z.B. Alkalihydride wie Natriumhydrid; Alkoholate wie Natrium- oder Kaliummethoxid oder -ethoxid, Aluminiumethoxid oder -isopropoxid; Oxide wie Calciumoxid, Bariumoxid oder Bleioxid oder Mineralsäuren wie Schwefelsäure, Chlorwasserstoff oder Bromwasserstoff oder Lewis-Säure, beispielsweise Bortrifluorid, Bortrichlorid und Brotrifluorid-Etherat oder organische Säuren wie p-Toluolsulfonsäure oder Trifluormethylsulfonsäure.

Das dabei freiwerdende Methanol wird aus dem Reaktionsgemisch destillativ oder mit Hilfe eines Inertgases wie Stickstoff oder Argon entfernt.

Es wurde ferner gefunden, daß man die neuen Verbindungen erhält,

3. wenn man ein Dibenzofuranoxyfettsäure-Metallsalz der Formel

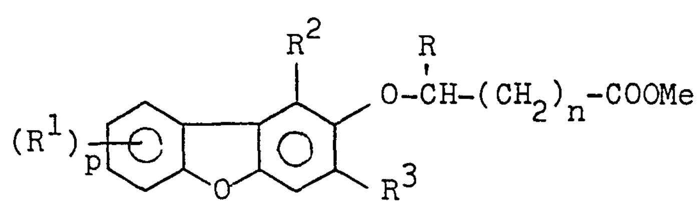

in welcher $R^1$, $R^2$, $R^3$, R, Me, n und p die oben angegebenen Bedeutungen haben, mit einem Aryloxy- oder Arylthio-alkyl-

halogenid der Formel

$$Z\text{-}(CH_2)_m\text{-}Y\text{-}Ar$$

in welcher
Ar, Y, Z und m die oben angegebenen Bedeutungen haben, gegebenenfalls in Gegenwart eines Lösungs- oder Verdünnungsmittels und gegebenenfalls unter Zusatz eines Reaktionsbeschleunigers, bei Temperaturen zwischen 0 und 150°C umsetzt.

Die Metallsalze sind aus der DE-OS 27 32 924 und DE-OS 28 23 424 bekannt. Die Halogenide können leicht nach bekannten Verfahren hergestellt werden, beispielsweise durch Umsetzung von Phenolen bzw. von Thiophenolen mit aliphatischen Dihalogeniden - wie 1,2-Dibromethan, 1,3-Dibrompropan, 1-Chlor-3-brompropan oder 1,4-Dibrombutan bevorzugt in siedendem Aceton, Methylethylketon, Diethylketon oder Cyclopentanon in Gegenwart von mindestens äquivalenten Mengen Kaliumcarbonat (vgl. Houben-Weyl, Methoden der organischen Chemie, Band 6/3, S. 54-59, Thieme-Verlag, Stuttgart 1965).

Als bevorzugte Lösungs- und Verdünnungsmittel für das Verfahren 3 werden z.B. Alkohole wie Methanol, Ethanol, Propanol oder Isopropanol; Ketone wie Aceton, Methylethylketon oder Diethylketon; Ester wie Essigsäureethylester; Ether wie Dimethoxyethan oder Dioxan; Nitrile wie Acetonitril; Sulfoxide wie Dimethylsulfoxid oder Amide wie Diemthylformamid, Dimethylacetamid oder N-Methylpyrrolidon oder entsprechende Gemische verwendet.

Als Reaktionsbeschleuniger werden vorzugsweise Metallhalogenide wie Natriumbromid, Natriumjodid oder Kaliumjodid oder Kronenether wie 18-Krone-6 und Dibenzo-18-krone-6 verwendet.

Es wurde schließlich gefunden, daß weitere neue Verbindungen durch nachträgliche Einführung der Substituenten $R^2$ un $R^3$ erhalten werden können. So kann man beispielsweise eine Untergruppe der neuen Verbindungen herstellen,

4. indem man Verbindungen der Formel

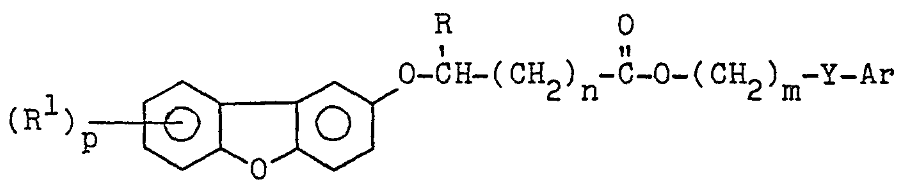

in welcher
$R^1$, R, Y, Ar, m, n und p die oben angegebenen Bedeutungen haben,

a) bromiert oder
b) chloriert oder
c) nitriert.

Die Mono- und Dibromierung bzw. -chlorierung werden beispielsweise mit Brom, N-Bromsuccinimid, Chlor, N-Chlorsuccinimid, Sulfurylchlorid oder anderen üblichen Bromierungs- bzw. Chlorierungsmitteln gegebenenfalls in Gegenwart eines Lösungs- und Verdünnungsmittels, gegebenenfalls in Gegenwart eines Katalysators und gegebenenfalls in Gegenwart eines organischen oder anorganischen Säure-bindenden Mittels, bei Temperaturen von -10 bis 120°C durchgeführt.

Als gegenüber den Reaktionsteilnehmern inerten Lösungs- und Verdünnungsmittel werden bevorzugt organische Säuren wie Essigsäure, Chloressigsäure, Trichloressigsäure und Trifluoressigsäure oder Halogenkohlenwasserstoffe wie Methylenchlorid, Chloroform, Tetrachlorkohlenstoff, 1,2--Dichlorethan, Chlorbenzol und Dichlorbenzol verwendet.

Geeignete Säure-bindende Mittel sind beispielsweise Natrium- oder Kaliumacetat. Als Katalysatoren eignen sich z.B. Lewis-Säure wie Jod, Zinkchlorid, Eisenchlorid, Zinntetrachlorid, Antimontribromid, Antimonpentachlorid, Bortribromid, Bortrichlorid, Aluminiumbromid oder Aluminiumchlorid oder Sulfide wie Diphenylsulfid oder Diphenyldisulfid.

Die Nitrierung der Verbindungen wird vorzugsweise mit Salpetersäure oder Metallnitraten wie beispielsweise Natrium- oder Kaliumnitrat gegebenenfalls in Gegenwart von organischen Säuren wie Essigsäure oder deren Anhydriden oder von Mineralsäuren wie Schwefelsäure oder Fluorborsäure, bei Temperaturen von 0 bis 100°C durchgeführt.

Das Verfahren 1a zur Herstellung der neuen Verbindungen wird bevorzugt.

Die neuen Verbindungen besitzen je nach der Beschaffenheit von R ein chirales Kohlenstoffatom mit H, R, 3-Dibenzofuranoxy- und $-(CH_2)_n-\overset{O}{\overset{\|}{C}}-O-(CH_2)_m-Y-Ar$ als Liganden. Nach üblichen Methoden können die optisch reinen Enantiomeren erhalten wer en. Die vorliegende Erfindung erfaßt auch diese Verbindungen, d.h. die neuen Enantiomeren und ihre Gemische. Als Herbizide kann man sowohl die reinen Enantiomeren als auch die bei der Synthese üblicherweise anfallenden Racemate verwenden.

Als Beispiele für die neuen erfindungsgemäßen Wirkstoffe seien im folgenden einzelne Verbindungen genannt:

7-Fluor-3-dibenzofuranoxyessigsäure-2'-phenoxyethylester
7-Fluor-2-nitro-3-dibenzofuranoxyessigsäure-2'-phenoxyethylester
7-Fluor-3-dibenzofuranoxyessigsäure-3'-phenoxypropylester
7-Fluor-3-dibenzofuranoxyessigsäure-2'-(3-chlorphenoxy)-ethylester
7-Fluor-3-dibenzofuranoxyessigsäure-2'-(4-chlorphenoxy)-ethylester
7-Fluor-3-dibenzofuranoxyessigsäure-4'-(3-trifluormethyl-phenoxy)-butylester
7-Fluor-3-dibenzofuranoxyessigsäure-2'-(3-methyl-phenoxy)-ethylester
2-(7-Fluor-3-dibenzofuranoxy)-propionsäure-2'-phenoxyethylester
2-(7-Fluor-3-dibenzofuranoxy)-propionsäure-2'-(3-chlorphenoxy)-ethylester
2-(7-Fluor-3-dibenzofuranoxy)-propionsäure-2'-(4-chlorphenoxy)-ethylester
2-(7-Fluor-3-dibenzofuranoxy)-propionsäure-2'-(4-chlorphenylthio)-ethylester
2-(7-Fluor-2-nitro-3-dibenzofuranoxy)-propionsäure-2'-(4-nitrophenoxy)-ethylester
2-(7-Fluor-3-dibenzofuranoxy)-propionsäure-2'-(3-methylphenoxy)-ethylester
2-(7-Fluor-3-dibenzofuranoxy)-propionsäure-3'-(3-chlorphenoxy)-propylester
2-(7-Fluor-3-dibenzofuranoxy)-propionsäure-2'-(4-bromphenoxy)-ethylester
4-(7-Fluor-3-dibenzofuranoxy)-buttersäure-2'-phenoxyethylester
4-(7-Fluor-3-dibenzofuranoxy)-buttersäure-2'-(3-chlorphenoxy)-ethylester

4-(7-Fluor-3-dibenzofuranoxy)-buttersäure-2'-(3-methylphenoxy)-ethylester

4-(7-Fluor-3-dibenzofuranoxy)-buttersäure-4'-phenoxybutylester

4-(7-Fluor-3-dibenzofuranoxy)-buttersäure-3'-(3-trifluormethyl-phenoxy)-propylester

2-(7-Fluor-3-dibenzofuranoxy)-buttersäure-2'-phenoxyethylester

2-(7-Fluor-3-dibenzofuranoxy)-buttersäure-3'-phenoxypropylester

2-(7-Fluor-3-dibenzofuranoxy)-buttersäure-2'-(3-chlorphenoxy)-ethylester

2-(2,4-Dichlor-7-fluor-3-dibenzofuranoxy)-buttersäure-2'-(2,4-dichlorphenoxy)-ethylester

7-Chlor-3-dibenzofuranoxyessigsäure-2'-phenoxyethylester

7-Chlor-3-dibenzofuranoxyessigsäure-3'-phenoxypropylester

7-Chlor-3-dibenzofuranoxyessigsäure-2'-(3-chlorphenoxy)-ethylester

7-Chlor-3-dibenzofuranoxyessigsäure-2'-(4-chlorphenoxy)-ethylester

2,3,7-Trichlor-3-dibenzofuranoxyessigsäure-2'-(2,4-dichlorphenoxy)-ethylester

7-Chlor-2-nitro-3-dibenzofuranoxyessigsäure-2'-(4-nitrophenoxy)-ethylester

7-Chlor-3-dibenzofuranoxyessigsäure-4'-(3-trifluormethylphenoxy)-butylester

7-Chlor-3-dibenzofuranoxyessigsäure-2'-(4-biphenyloxy)-ethylester

2-(7-Chlor-3-dibenzofuranoxy)-propionsäure-2'-(3-dibenzofuranoxy)-ethylester

2-(7-Chlor-3-dibenzofuranoxy)-propionsäure-2'-(1-naphthyloxy)-ethylester

2-(7-Chlor-3-dibenzofuranoxy)-propionsäure-2'-(2-naphthyloxy)-ethylester

2-(7-Chlor-3-dibenzofuranoxy)-propionsäure-2'-phenoxyethylester

2-(7-Chlor-3-dibenzofuranoxy)-propionsäure-3'-phenoxy-propylester

7-(7-Chlor-3-dibenzofuranoxy)-propionsäure-4'-phenoxybutyl-ester

2-(7-Chlor-3-dibenzofuranoxy)-propionsäure-2'-(3-chlorphen-oxy)-ethylester

2-(7-Chlor-3-dibenzofuranoxy)-propionsäure-2'-(4-chlor-phenylthio)-ethylester

2-(7-Chlor-3-dibenzofuranoxy)-propionsäure-2'-(4-bromphenoxy)-ethylester

2-(7-Chlor-3-dibenzofuranoxy)-propionsäure-2'-(4-bromphenyl-thio)-ethylester

2-(7-Chlor-3-dibenzofuranoxy)-propionsäure-2'-(3-methylphen-oxy)-ethylester

2-(7-Chlor-3-dibenzofuranoxy)-propionsäure-2'-(2-methyl--4-chlorphenoxy)-ethylester

2-(7-Chlor-3-dibenzofuranoxy)-propionsäure-2'-(2,4-dimethyl-phenoxy)-ethylester

2-(7-Chlor-3-dibenzofuranoxy)-propionsäure-2'-(3-trifluormethyl-phenoxy)-ethylester

2-(7-Chlor-3-dibenzofuranoxy)-propionsäure-4'-(3-trifluormethyl-phenoxy)-butylester

2-(7-Chlor-3-dibenzofuranoxy)-propionsäure-4'-(4-chlor-phenoxy)-butylester

2-(7-Chlor-3-dibenzofuranoxy)-propionsäure-2'-(3-methoxy-4-methylphenoxy)-ethylester

2-(7-Chlor-3-dibenzofuranoxy)-propionsäure-2'-(2,4-di-chlorphenoxy)-ethylester

2-(7-Chlor-3-dibenzofuranoxy)-propionsäure-2'-(3,5-di-chlorphenoxy)-ethylester

2-(7-Chlor-3-dibenzofuranoxy)-propionsäure-2'-(2,5-dichlor-phenoxy)-ethylester

2-(7-Chlor-3-dibenzofuranoxy)-propionsäure-2'-(3,4-dichlor-phenoxy)-ethylester

2-(7-Chlor-3-dibenzofuranoxy)-propionsäure-2'-(4-cyan-phenoxy)-ethylester

2-(7-Chlor-3-dibenzofuranoxy)-propionsäure-2'-(2-methoxy--4-allylphenoxy)-ethylester
2-(7-Chlor-3-dibenzofuranoxy)-propionsäure-2'-(4-ethoxy-phenoxy)-ethylester
2-(7-Chlor-3-dibenzofuranoxy)-propionsäure-2'-(2,4,6-tri-chlorphenoxy)-ethylester
2-(7-Chlor-3-dibenzofuranoxy)-propionsäure-2'-(2,4,5-tri-chlorphenoxy)-ethylester
2-(2,4,7-Trichlor-3-dibenzofuranoxy)-propionsäure-2'-phen-oxyethylester
2-(2,4,7-Trichlor-3-dibenzofuranoxy)-propionsäure-2'-(3--chlorphenoxy)-ethylester
2-(2,4,7-Trichlor-3-dibenzofuranoxy)-propionsäure-3-phen-oxypropylester
4-(7-Chlor-3-dibenzofuranoxy)-buttersäure-2'-phenoxyethyl-ester
4-(7-Chlor-3-dibenzofuranoxy)-buttersäure-2'-(3-chlorphen-oxy)-ethylester
4-(7-Chlor-3-dibenzofuranoxy)-buttersäure-3'-(3-chlorphen-oxy)-propylester
4-(7-Chlor-3-dibenzofuranoxy)-buttersäure-2'-(4-methyl-phenoxy)-ethylester
7-Brom-3-dibenzofuranoxyessigsäure-2'-phenoxyethylester
7-Brom-3-dibenzofuranoxyessigsäure-2'-(3-chlorphenoxy)--ethylester
7-Brom-3-dibenzofuranoxyessigsäure-2'-(3-trifluormethyl--phenoxy)-ethylester
2-(7-Brom-3-dibenzofuranoxy)-propionsäure-2'-phenoxyethyl-ester
2-(7-Brom-3-dibenzofuranoxy)-propionsäure-2'-(3-chlorphen-oxy)-ethylester
2-(7-Brom-3-dibenzofuranoxy)-propionsäure-4'-(3-trifluormethyl-phenoxy)-butylester
2-(7-Brom-3-dibenzofuranoxy)-propionsäure-2'-(3-methyl-phenoxy)-ethylester

2-(7-Brom-3-dibenzofuranoxy)-propionsäure-2'-(4-chlorphenylthio)-ethylester
2-(7-Brom-3-dibenzofuranoxy)-propionsäure-2'-(2-methyl-4-methylthio-phenoxy)-ethylester
2-(7-Brom-3-dibenzofuranoxy)-propionsäure-2'-(2-methoxy-4-acetyl-phenoxy)-ethylester
2-(2-Nitro-7-brom-3-dibenzofuranoxy)-propionsäure-2'-(3-chlorphenoxy)-ethylester
2-(2,4,7-Tribrom-3-dibenzofuranoxy)-propionsäure-2'-(4-bromphenoxy)-ethylester
2-(7-Chlor-3-dibenzofuranoxy)-buttersäure-3'-phenoxypropylester
2-(7-Chlor-3-dibenzofuranoxy)-buttersäure-2'-(3-chlorphenoxy)-ethylester
2-(7-Chlor-3-dibenzofuranoxy)-buttersäure-2'-(3-methylphenoxy)-ethylester
2-(7-Chlor-3-dibenzofuranoxy)-buttersäure-2'-(3-trifluormethyl-phenoxy)-ethylester
7-Methyl-3-dibenzofuranoxyessigsäure-2'-phenoxyethylester
2-(7-Methyl-3-dibenzofuranoxy)-propionsäure-2'-(3-chlorphenoxy)-ethylester
2-(7-Methyl-3-dibenzofuranoxy)-buttersäure-2'-(3-methylphenoxy)-butylester
2-(6,7-Dichlor-3-dibenzofuranoxy)-propionsäure-2'-phenoxyethylester
2-(6,7-Dichlor-3-dibenzofuranoxy)-propionsäure-3'-phenoxypropylester
2-(6,7-Dichlor-3-dibenzofuranoxy)-propionsäure-2'-(3-chlorphenoxy)-ethylester
2-(6,7-Dichlor-3-dibenzofuranoxy)-propionsäure-2'-(3-methylphenoxy)-ethylester
7-Trifluormethyl-3-dibenzofuranoxyessigsäure-2'-phenoxyethylester
7-Trifluormethyl-3-dibenzofuranoxyessigsäure-2'-(3-chlorphenoxy)-ethylester

7-Trifluormethyl-3-dibenzofuranoxyessigsäure-4'-(3-trifluormethyl-phenoxy)-butylester

2-(7-Trifluormethyl-3-dibenzofuranoxy)-propionsäure-2'-phenoxyethylester

2-(7-Trifluormethyl-3-dibenzofuranoxy)-propionsäure-3'-phenoxypropylester

2-(7-Trifluormethyl-3-dibenzofuranoxy)-propionsäure-4'-phenoxybutylester

2-(7-Trifluormethyl-3-dibenzofuranoxy)-propionsäure-2'-(3-chlorphenoxy)-ethylester

2-(7-Trifluormethyl-3-dibenzofuranoxy)-propionsäure-2'-(3-methylphenoxy)-ethylester

2-(7-Trifluormethyl-3-dibenzofuranoxy)-propionsäure-2'-(3-trifluormethyl-phenoxy)-ethylester

2-(7-Trifluormethyl-3-dibenzofuranoxy)-propionsäure-4'-(3-trifluormethyl-phenoxy)-butylester

2-(7-Trifluormethyl-3-dibenzofuranoxy)-propionsäure-2'-(4-chlorphenylthio)-ethylester

2-(7-Trifluormethyl-3-dibenzofuranoxy)-propionsäure-2'-(2,4,6-trimethyl-phenoxy)-ethylester

2-(7-Trifluormethyl-3-dibenzofuranoxy)-buttersäure-2'-(3-chlorphenoxy)-ethylester

2-(7-Trifluormethyl-3-dibenofuranoxy)-buttersäure-2'-(3-methylphenoxy)-ethylester

2-(2,4-Dichlor-7-trifluormethyl-3-dibenzofuranoxy)-propionsäure-2'-phenoxyethylester

4-(7-Trifluormethyl-3-dibenzofuranoxy)-valeriansäure-2'-phenoxyethylester

4-(7-Trifluormethyl-3-dibenzofuranoxy)-valeriansäure-3'-phenoxypropylester

4-(7-Trifluormethyl-3-dibenzofuranoxy)-valeriansäure-2'-(3-chlorphenoxy)-ethylester

4-(7-Trifluormethyl-3-dibenzofuranoxy)-valeriansäure-2'-(3-methylphenoxy)-ethylester

2-(7-Tetrafluromethoxy-3-dibenzofuranoxy)-propionsäure-2'-phenoxyethylester

2-(7-Tetrafluorethoxy-3-dibenzofuranoxy)-propionsäure-2'-
-(2-chlorphenoxy)-ethylester
2-(7-Tetrafluorethoxy-3-dibenzofuranoxy)-propionsäure-2'-
-(3-methylphenoxy)-ethylester

Die Herstellung der Wirkstoffe wird durch folgende Beispiele erläutert.

Beispiel 1

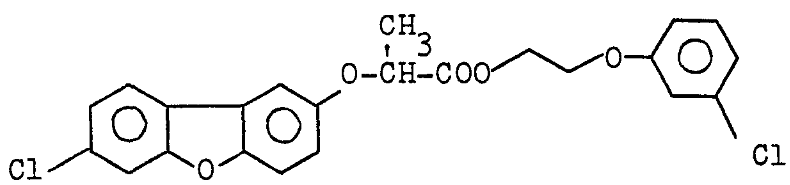

a) 890 g (6,45 Mol) trockenes Kaliumcarbonat werden in einer Lösung von 645 g (2,53 Mol) 2',4'-Dichlor-2,5-dihydroxybiphenyl in 2000 ml Dimethylformamid suspendiert. Nach 20-stündigem Rühren bei 145-150°C unter Stickstoff bildet sich quantitativ das 7-Chlor-3-dibenzofuranol. Das Reaktionsgemisch wird auf 85°C abgekühlt und innerhalb von 8 Stunden mit 870,2 g (2,83 Mol) 2-Brompropionsäure-2'-(3-chlorphenoxy)-ethylester gelöst in 500 ml Dimethylformamid tropfenweise versetzt. Nach 10-stündigem Nachrühren bei 85°C wird das Gemisch warm abgesaugt und der anorganische Niederschlag mit 200 ml Dimethylformamid gewaschen. Das Filtrat wird im Vakuum eingeengt. Der Rückstand wird mit 400 ml Methanol versetzt, bei 10°C ca. 30 Minuten gerührt, abgesaugt und der kristalline Niederschlag mit 200 ml kaltem Methanol gewaschen und getrocknet. Man erhält 923,2 g (82 % d.Th.) 2-(7-Chlor-3-dibenzofuranoxy)-propionsäure-2'-(3-chlorphenoxy)-ethylester vom Schmelzpunkt 95-97°C (Wirkstoff Nr. 1).

b) 41,4 g (0,3 Mol) trockenes Kaliumcarbonat werden in einer Lösung von 55,3 g (0,253 Mol) 7-Chlor-3-dibenzofuranol in 200 ml Methylethylketon suspendiert. Das Gemisch wird auf 75°C erwärmt und innerhalb von 8 Stunden mit 87 g (0,283 Mol) 2-Brompropionsäure-2'-(3--chlorphenoxy)-ethylester gelöst in 100 ml Methylethylketon tropfenweise versetzt. Nach zehnstündigem Nachrühren bei 80°C wird das Gemisch abgekühlt, abgesaugt und der anorganische Niederschlag mit 100 ml Methylethylketon gewaschen. Das Filtrat wird im Vakuum eingeengt. Der Rückstand wird mit einem Gemisch von 50 ml Diethylether und 50 ml Petrolether gerührt, abgesaugt und getrocknet. Man erhält 102,4 g (91 % d.Th.) Produkt vom Schmelzpunkt 96-97°C.

c) 29 g (0,1 Mol) 2-(7-Chlor-3-dibenzofuranoxy)-propionsäure, 19 g (0,11 Mol) 2-(3-Chlorphenoxy)-ethanol, 0,5 ml konz. Schwefelsäure und 300 ml Toluol werden 8 Stunden am Wasserabscheider bei 110°C gerührt bis 1,8 g Wasser entfernt sind. Das Gemisch wird zweimal mit je 100 ml Wasser gewaschen, auf 90 ml eingeengt, abgekühlt und abgesaugt. Der Niederschlag wird mit 10 ml kaltem Methanol, dann mit 200 ml Wasser neutral gewaschen und getrocknet. Man erhält 42,3 g (95 % d.Th.) Produkt vom Schmelzpunkt 95-97°C.

d) Eine Lösung von 12,1 g (0,07 Mol) 2-(3-Chlorphenoxy)--ethanol und 7,5 g (0,075 Mol) Triethylamin in 200 ml Toluol wird mit einer Lösung von 20,4 g (0,065 Mol) 2-(7-Chlor-3-dibenzofuranoxy-propionsäurechlorid bei 15°C tropfenweise versetzt. Das Gemisch wird über Nacht bei Raumtemperatur gerührt und abgesaugt. Das Filtrat wird zweimal mit je 50 ml Wasser gewaschen, über $Na_2SO_4$ getrocknet und eingeengt. Der Rückstand wird mit einem

Gemisch von 30 ml Diethylether und 30 ml Petrolether gerührt, abgesaugt und getrocknet. Man erhält 22 g (76 % d.Th.) Produkt vom Schmelzpunkt 96-97°C.

e) 30,4 g (0,1 Mol) 2-(7-Chlor-3-dibenzofuranoxy)-propionsäuremethylester und 86,2 g (0,5 Mol) 2-(3-Chlorphenoxy)-ethanol werden mit 1,1 g (0,02 Mol) Natriummethoxid versetzt und bei 135 bis 145°C 8 Stunden in einem schwachen Stickstoffstrom gerührt. Das Gemisch wird abgekühlt, mit 300 ml Methylenchlorid verdünnt und einmal mit 100 ml Wasser, dann mit 50 ml 1n Salzsäure und schließlich mit 100 ml Wasser gewaschen, getrocknet und vom Dimethylether durch Eindampfen im Vakuum befreit. Der Überschuß von 2-(3-Chlorphenoxy)--ethanol wird bei 110°C im Vakuum (0,02 mm Hg) abdestilliert. Der Rückstand wird mit einem Gemisch von 50 ml Diethylether und 30 ml Petrolether gerührt, abgesaugt und getrocknet. Man erhält 41,8 g (94 % d.Th.) Produkt vom Schmelzpunkt 94-97°C.

Herstellung der Ausgangsverbindung

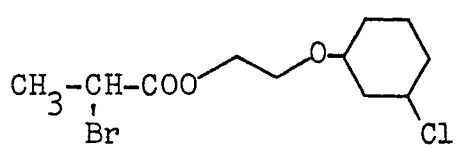

167 g (1 Mol) 2-Brompropionsäuremethylester werden in Gegenwart von 2 g konz. Schwefelsäure mit 163,9 g (0,95 Mol) 2-(3-Chlorphenoxy)-ethanol nach Rinehart (Org. Synth. Coll. Vol. IV, 122 (1963)) umgeestert. Ausbeute 219,1 g (75 % d.Th.) 2-Brompropionsäure-2'-(3-Chlorphenoxy)-ethylester vom Siedepunkt 106°C/1 mm Hg und Schmelzpunkt 32°C.

Beispiel 2

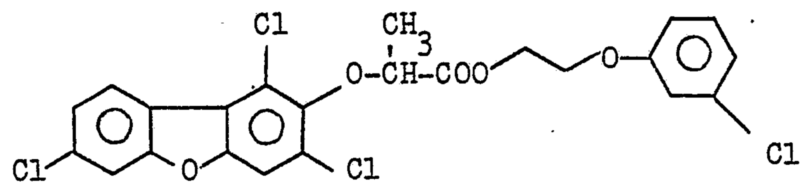

Eine Lösung von 44,5 g (0,1 Mol) 2-(7-Chlor-3-dibenzofuranoxy)-propionsäure-2'-(3-chlorphenoxy)-ethylester in 300 ml Chlorbenzol wird mit 1 g Jod und mit 33,6 g (0,24 Mol) Sulfurylchlorid versetzt und 8 Stunden bei 70°C gerührt. Das Gemisch wird abgekühlt, zweimal mit je 200 ml Wasser, einmal mit 150 ml 10 %iger Natriumbisulfitlösung und wieder mit 100 ml Wasser gewaschen, getrocknet und im Vakuum eingeengt. Der Rückstand wird mit 100 ml Petrolether gerührt, abgesaugt, mit Petrolether gewaschen und getrocknet. Man erhält 40,6 g (79 % d.Th.) 2-(2,4,7-Trichlor-3-dibenzofuranoxy)-propionsäure-2'-(3-chlorphenoxy)-ethylester vom Schmelzpunkt 73-75°C (Wirkstoff Nr. 2).

Beispiel 3

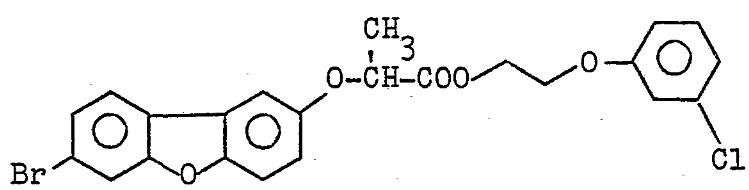

Eine Lösung von 14 g (0,0376 Mol) 2-(7-Brom-3-dibenzofuranoxy)-propionsäure-Kaliumsalz in 80 ml Dimethylformamid wird mit der Lösung von 9,1 g (0,038 Mol) 2-(3-Chlorphenoxy)-bromethan in 30 ml Dimethylformamid versetzt und 12 Stunden bei 100°C gerührt. Das Gemisch wird im Vakuum eingeengt. Der Rückstand wird zwischen 50 ml Wasser und 100 ml Ether verteilt, die organische Schicht dreimal mit je 50 ml Wasser gewaschen, mit Tierkohle entfärbt und im

Vakuum eingeengt. Der Rückstand wird mit Petrolether gerührt, abgesaugt und getrocknet. Man erhält 10 g (54,3 % d.Th.) 2-(7-Brom-3-dibenzofuranoxy)-propionsäure-2'-(3-chlorphenoxy)-ethylester als weiße Kristalle vom Schmelzpunkt 86-88°C (Wirkstoff Nr. 3).

Beispiel 4

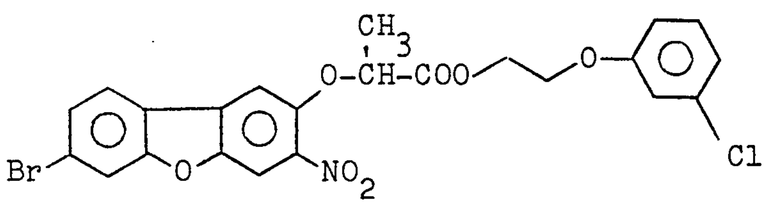

Eine Lösung von 12,2 g (0,025 Mol) 2-(7-Brom-3-dibenzofuranoxy)-propionsäure-2'-(3-chlorphenoxy)-ethylester in 90 ml Chlorbenzol wird mit 5 ml (0,065 Mol) 65 %iger Salpetersäure versetzt und 8 Stunden bei 60 bis 70°C gerührt und über Nacht (12 Stunden) bei Raumtemperatur (20°C) stehen gelassen. Das Gemisch wird auf 10°C abgekühlt und der Niederschlag abgesaugt, mit 15 ml kaltem Monochlorbenzol und mit 15 ml kaltem Methanol gewaschen. Der kristalline Niederschlag wird in 250 ml Methylenchlorid gelöst, mit 50 ml 1n NaOH, dann dreimal mit je 50 ml Wasser gewaschen, getrocknet und im Vakuum eingeengt. Der Rückstand wird mit 50 ml Petroläther gerührt, abgesaugt und getrocknet. Man erhält 11,5 g (86,3 % d.Th.) 2-(2-Nitro-7-brom-3-dibenzofuranoxy)-propionsäure-2'-(3--chlorphenoxy)-ethylester als hell-gelbes kristallines Pulver vom Schmelzpunkt 162-164°C (Wirkstoff Nr. 4).

Entsprechend Beispiel 1 bis 4 wurden folgende Verbindungen hergestellt, deren Strukturen durch $^{1}$H-Kernresonanz und IR-Spektren gesichert wurden.

5 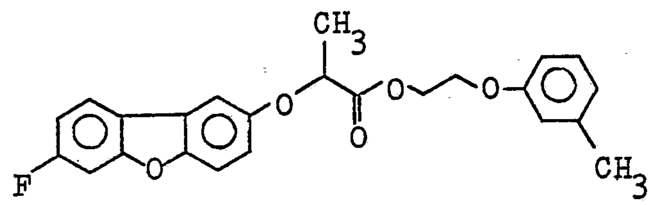 $n_D^{20}$ 1.5770

6 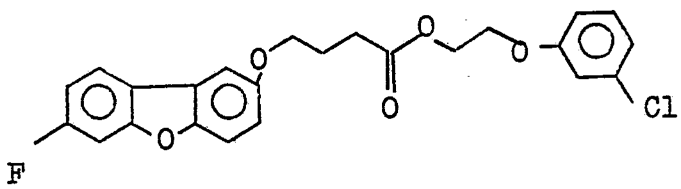 $n_D^{20}$ 1.5890

7 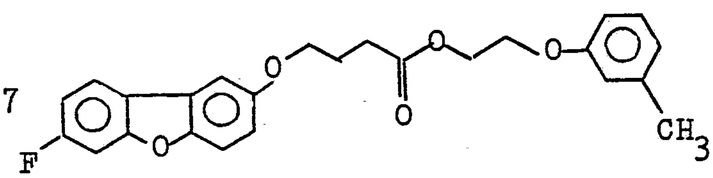 Wachs

8 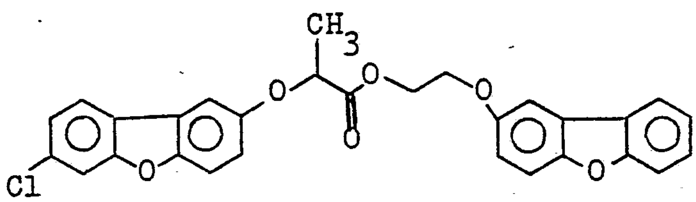 Fp. 104-106°C

9 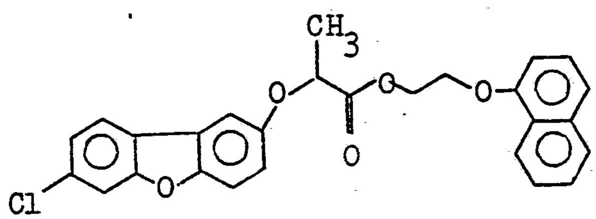 Fp. 82-83°C

10 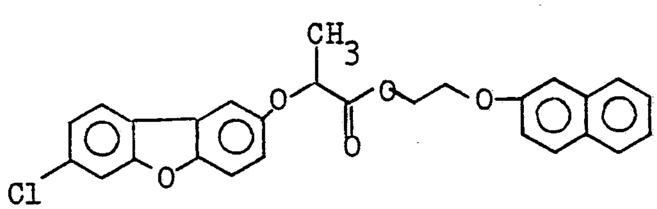 Fp. 145-147°C

11 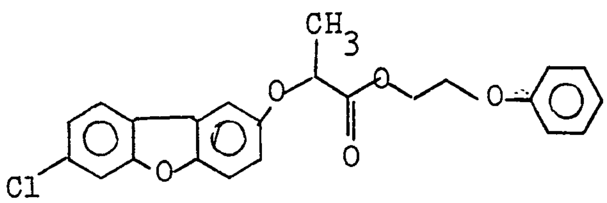 Fp. 85-87°C

12 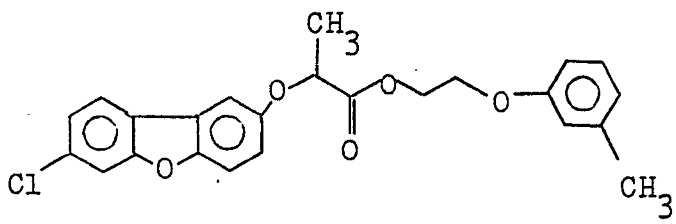 Fp. 92-94°C

13 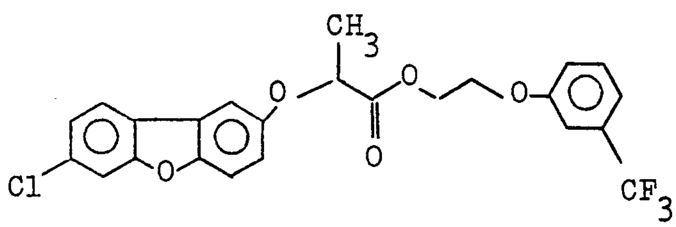 Fp. 41-44°C

14 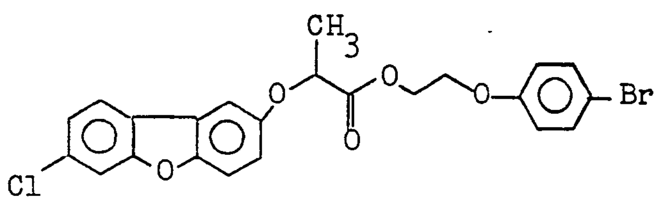 Fp. 72-75°C

15 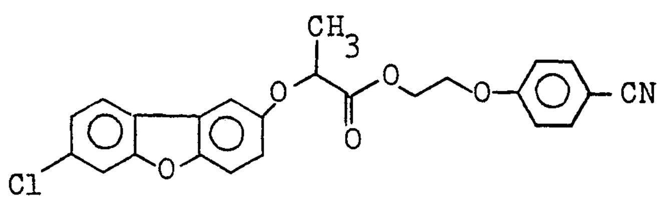 Fp. 109-111°C

16 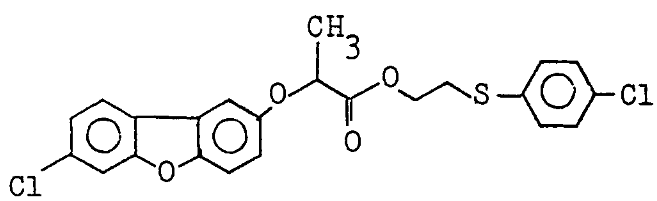 Fp. 67-69°C

17 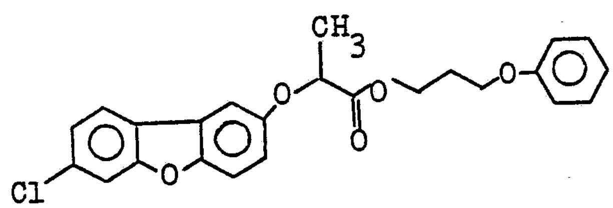 Fp. 83-85°C

18 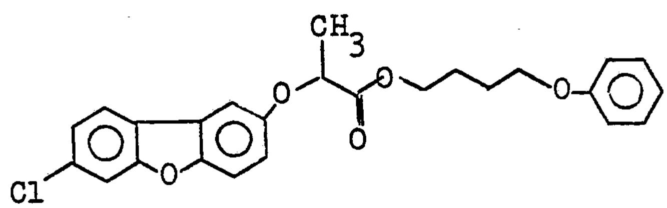 Fp. 77-80°C

19 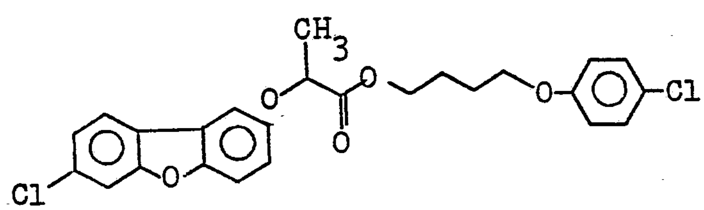 Fp. 91-94°C

20 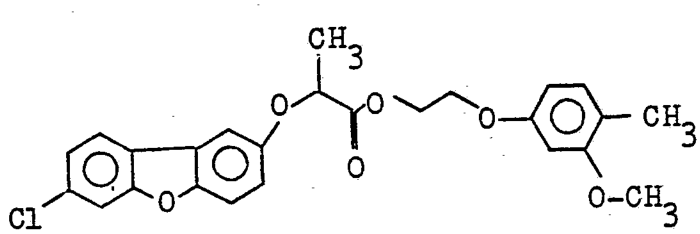 Fp. 80-83°C

21 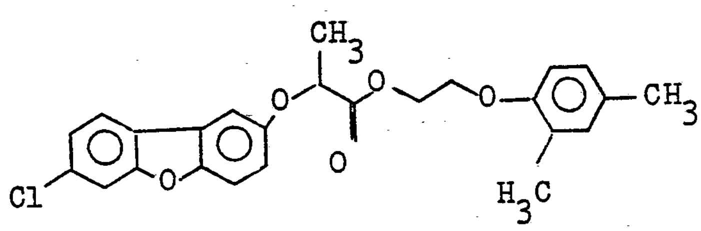 Fp. 90-91°C

22 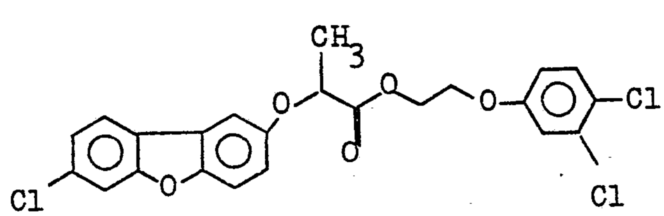 Fp. 124-126°C

23 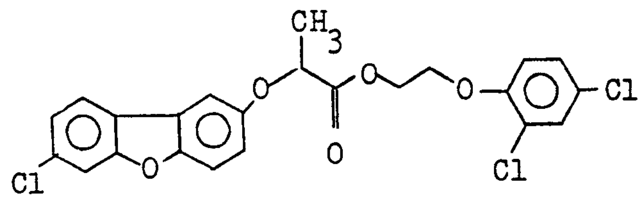 Fp. 128-130°C

24 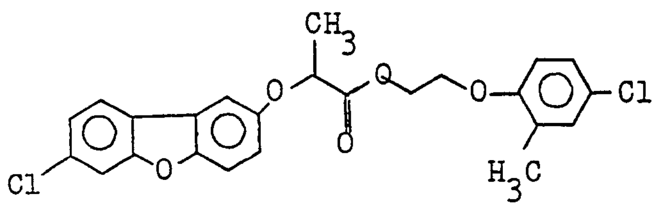 Fp. 124-127°C

25 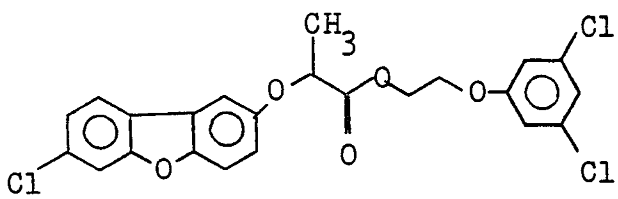 Fp. 75-78°C

26 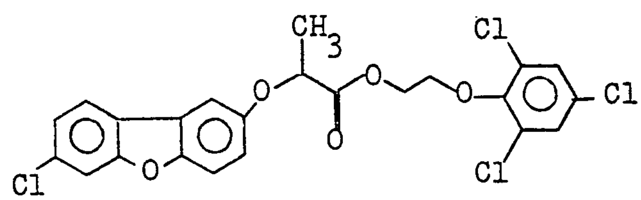 Fp. 133-135°C

27 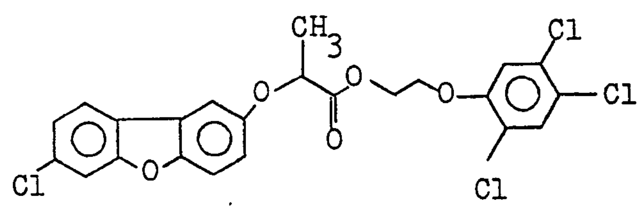 Fp. 122-123°C

28 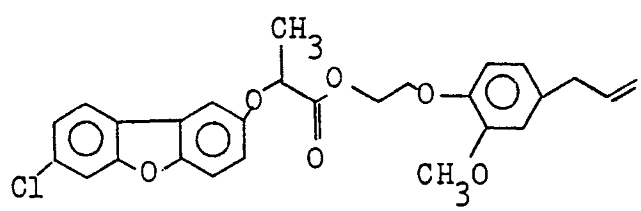 Fp. 101-103°C

29 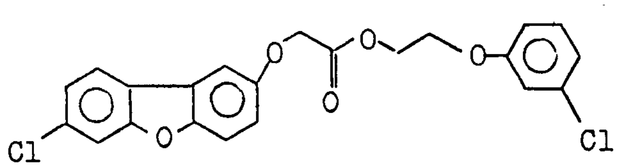 Fp. 134-136°C

30 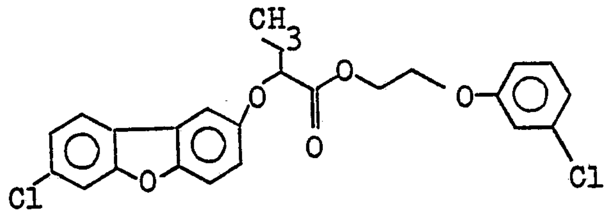 Fp. 54-56°C

31 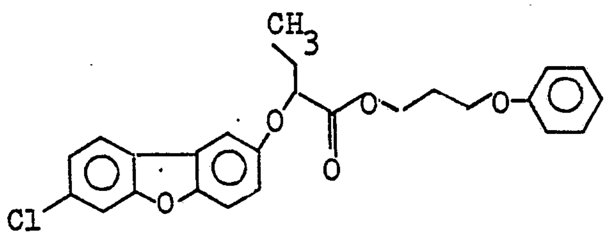 Fp. 68-70°C

32 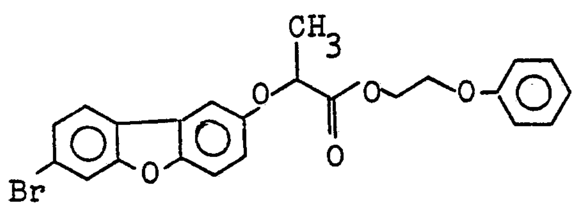 Fp. 71-73°C

33 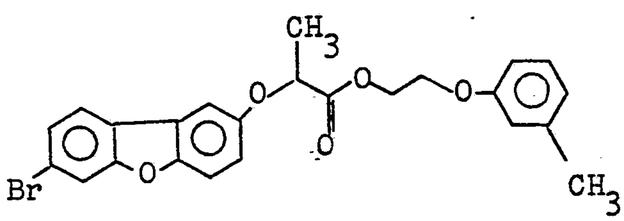 Fp. 79-81°C

34 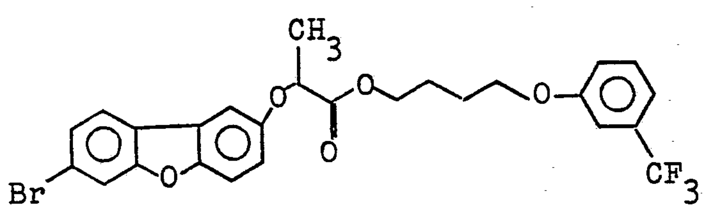 Harz

35 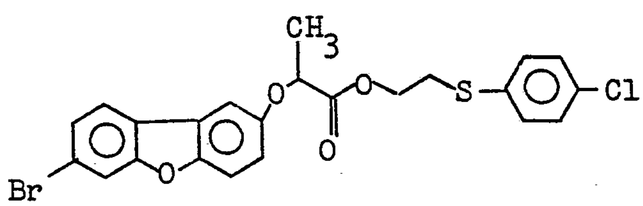 Öl

36
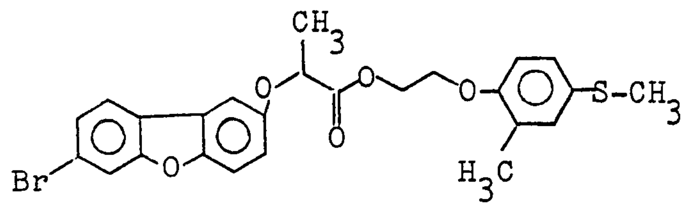
Fp. 68-72°C

37
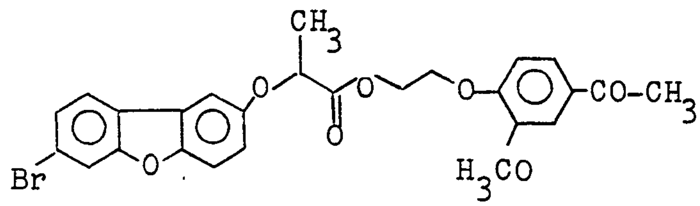
Fp. 148-150°C

38
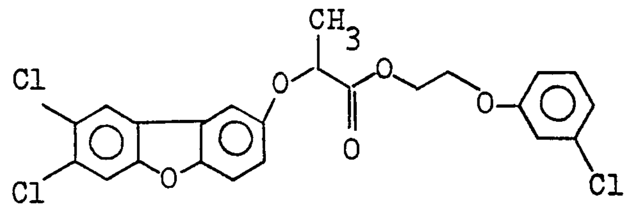
Fp. 73°C

39
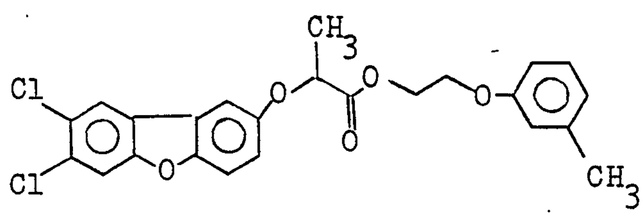
Fp. 57°C

40
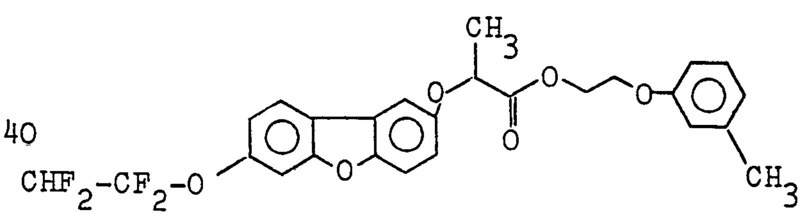
$n_D^{20}$ 1.5430

41
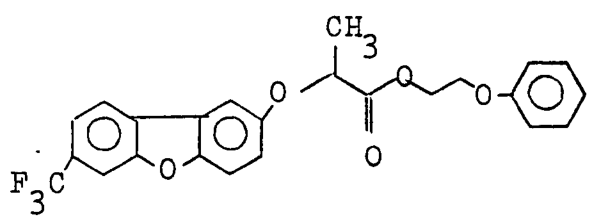
Öl

42 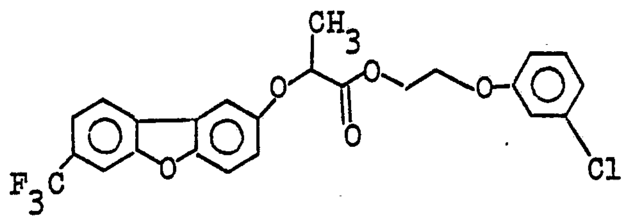 $n_D^{20}$ 1.5590

43 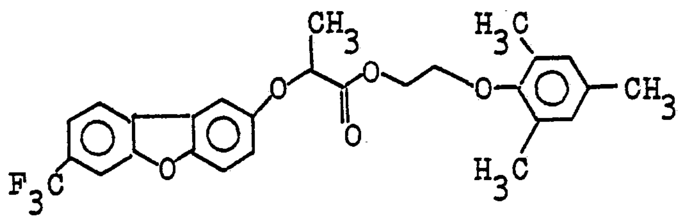 Öl

44 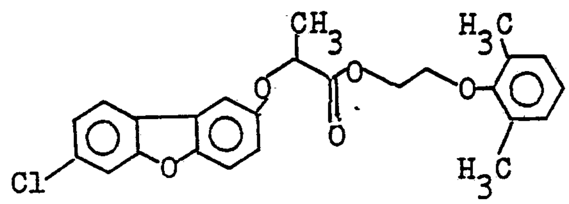 Fp. 73-75°C

45 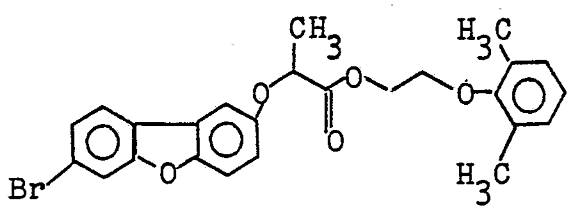 Fp. 69-72°C

46 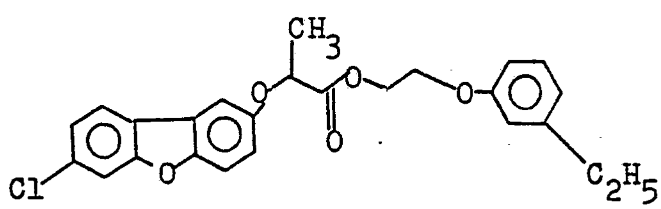 Fp. 71-73°C

47 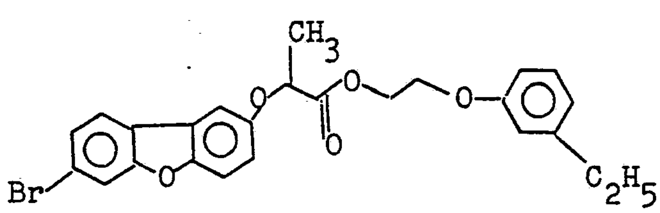 Fp. 69-71°C

48 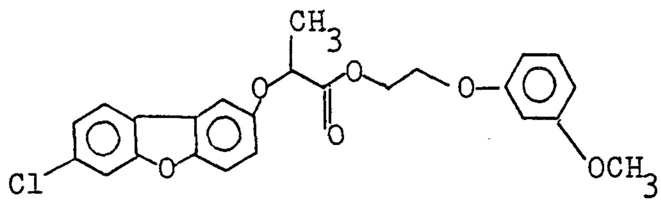 Fp. 60-62°C

49 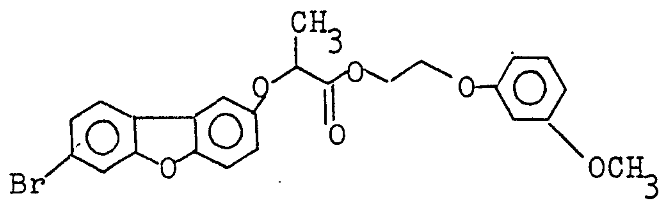 Fp. 65-67°C

Die Anwendung der Wirkstoffe erfolgt z.B. in Form von direkt versprühbaren Lösungen, Pulvern, Suspensionen - auch hochprozentige wässerige, ölige oder sonstige Suspensionen - oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem mit hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformid, Dimethylsulfoxid, N-Methylpyrrolidon, Wasser usw. in Betracht.

Wässerige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen: Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol-, Octylphenol-, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz und Nußschalenmehl, Cellulosepulver und andere feste Trägerstoffe.

Die Formulierungen enthalten zwischen 0,1 und 95 Gewichtsprozent Wirkstoff, vorzugsweise zwischen 0,5 und 90 Gewichtsprozent.

Die Wirkstoffe werden angewendet, beispielsweise durch Gießen, Streuen, Stäuben, Spritzen oder Sprühen auf die Pflanzen oder den Boden, durch Injizieren oder Bestreichen von Pflanzen oder durch Einbringen in das Bewässerungswasser.

Beispiel I

Man vermischt 90 Gewichtsteile der Verbindung 1 mit 10 Gewichtsteilen N-Methyl-alpha-pyrrolidon und erhält eine Lösung, die zur Anwendung in Form kleinster Tropfen geeignet ist.

Beispiel II

10 Gewichtsteile der Verbindung 2 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-mono-ethanolamid, 2 Gewichtsteilen Cal-

ciumsalz der Dodecylbenzolsulfonsäure und 2 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

Beispiel III

20 Gewichtsteile der Verbindung 2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel IV

20 Gewichtsteile der Verbindung 3 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel V

80 Gewichtsteile des Wirkstoffs 1 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 70 Gewichtsteilen pulverförmigem Kieselsäuregel gut vermischt und in einer Hammermühle vermahlen. Durch feines Verteilen der Mischung in Wasser erhält man eine Spritzbrühe.

Beispiel VI

95 Gewichtsteile der Verbindung 1 werden mit 5 Gewichtsteilen feinteiligem Kaolin innig vermischt. Man erhält auf diese Weise ein Stäubemittel, das 95 Gewichtsprozent des Wirkstoffs enthält.

Beispiel VII

30 Gewichtsprozent der Verbindung 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel VIII

40 Gewichtsteile des Wirkstoffs 1 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel IX

20 Teile des Wirkstoffs 1 werden mit 12 Teilen Calciumsalz der Dodecylbenzolsulfonsäure, 8 Teile Fettalkohol-polyglykolether, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

Die Applikation kann im Vorauflaufverfahren oder bei Nachauflaufanwendung erfolgen. Vorzugsweise werden die neuen Wirkstoffe nach dem Auflaufen der unerwünschten Pflanzen, sowohl auf Kulturflächen als auch auf unbebautem Land, ausgebracht. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen ans Wirkstoff betragen je nach Jahreszeit und Wachstumsstadium 0,1 bis 15 kg/ha und mehr.

Der Einfluß einiger Vertreter der neuen Dibenzofuranyloxyalkancarbonsäureester auf das Wachstum von unerwünschten Pflanzen- und Kulturgewächsen wird anhand von Gewächshausversuchen gezeigt:

Als Kulturgefäße dienten Plastikblumentöpfe mit 300 $cm^3$ Inhalt und lehmigem Sand mit etwa 1,5 % Humus als Substrat. Die Samen der Testpflanzen entsprechend Tabelle 1 wurden nach Arten getrennt flach eingesät, oder es wurden vorgekeimte Jungpflanzen oder Stecklinge eingesetzt.

Generell wurden die Pflanzen zum Zwecke der Nachauflaufbehandlung bis zu einer Höhe von 3 bis 10 cm gezogen und danach behandelt. Die Wirkstoffe wurden auf die Sproßteile der Pflanzen und die nicht völlig von Pflanzen abgedeckte Bodenfläche, in Wasser als Verteilungsmittel suspendiert oder emulgiert, mittels fein verteilender Düsen gespritzt. Die Versuchstöpfe wurden dann in verschiedenen Temperaturbereichen oder Gewächshausanlagen aufgestellt, wobei für wärmeliebende Arten 20 bis 30°C und für solche gemäßigter Klimate 10 bis 20°C bevorzugt wurden. Die Versuchsperiode erstreckte sich über 2 bis 4 Wochen. Während dieser Zeit wurden die Pflanzen gepflegt, und ihre Reaktion auf die einzelnen Wirkstoffe wurde ausgewertet. Die Auswertung der Versuche wurde nach einer Skala von 0 bis 100 vorgenommen. Dabei bedeutet 0 keine Schädigung und 100 völliges Absterben zumindest der oberirdischen Sproßteile.

Als Vertreter unerwünschter Pflanzen dienten folgende Arten:

| Lateinischer Name | Deutscher Name | Englischer Name |
|---|---|---|
| Galium aparine | Klettenlabkraut | Catchweed bedstraw |
| Euphorbia geniculata | Südamerik. Wolfsmilchart | Southamerican member of the spurge family |
| Sesbania exaltata | Turibaum | hemp sesbania (coffeeweed) |
| Sinapis alba | Weißer Senf | white mustard |
| Solanum nigrum | Schwarzer Nachtschatten | black nightshade |
| Ipomoea spp. | Prunkwinden | morning glories |

Aus der Reaktion dieser Unkräuter werden die herbiziden Wirkungen der Verbindungen ermittelt.

Als Kulturpflanzen sind beispielsweise vertreten:

| Lat. Name | Abkürzung in Tab. | Deutscher Name | Englischer Name |
|---|---|---|---|
| Oryza sativa | | Reis | rice |
| Triticum aestivum | Tritic. aestivum | Weizen | wheat |
| Zea mays | | Mais | Indian corn |
| Avena sativa | | Hafer | oats |

Als Ergebnis erkennt man die wesentlich bessere Kulturpflanzenverträglichkeit der neuen Dibenzofuranyloxyalkancarbonsäureester-Derivate in Relation zum Vergleichsmittel derselben Stoffklasse und ganz besonders krass im Vergleich zu dem eingesetzten Handelsprodukt.

In Anbetracht der guten Verträglichkeit der Wirkstoffe und der Vielseitigkeit der Applikationsmethoden können die neuen Verbindungen oder diese enthaltende Mittel außer bei den in Tabelle 1 aufgeführten Nutzpflanzen noch in einer weiteren großen Zahl von Kulturpflanzen zur Beseitigung unerwünschten Pflanzenwuchses eingesetzt werden.

In Betracht kommen beispielsweise die folgenden Kulturen:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fooder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weiße Rübe | turnips |
| Brassica rapa var. silvestris | Rübsen | |
| Camellia sinensis | Teestrauch | tea plants |
| Carthamus tinctorius | Saflor - Färberdistel | safflower |
| Cary illinoinensis | Pekannußbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |

| Botanischer Name | Deutscher Name | Englischer Name |
|---|---|---|
| Cucumis sativus | Gurke | cucumber |
| Cynodon dactylon | Bermudagras | Bermudagrass in turfs and lawn |
| Daucus carota | Möhre | carrots |
| Elaeis guineensis | Ölpalme | oil palms |
| Fragaria vesca | Erdbeere | strawberries |
| Glyoine max | Sojabohne | soybeans |
| Gossypium hirsutum (Gossypium arboreum Gossypium herbaceum Gossypium vitifolium) | Baumwolle | cotton |
| Helianthus annuus | Sonnenblume | sunflowers |
| Helianthus tuberosus | Topinambur | |
| Hevea brasiliensis | Parakatuschukbaum | rubber plants |
| Hordeum vulgare | Gerste | barley |
| Humulus lupulus | Hopfen | hop |
| Ipomoea batatas | Süßkartoffeln | sweet potato |
| Juglans regia | Walnußbaum | walnut trees |
| Lactua sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |

| Botanischer Name | Deutscher Name | Englischer Name |
|---|---|---|
| Manihot esculenta | Maniok | cassava |
| Medicago sativa | Luzerne | alfalfa (lucerne) |
| Metha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohrkolben-hirse | |
| Petroselinum crispum spp. tuberosum | Wurzelpetersilie | parsley |
| Picea abies | Rotfichte | Norway spruce |
| Abies alba | Weißtanne | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |

| Botanischer Name | Deutscher Name | Englischer Name |
|---|---|---|
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sasamum indicum | Sesam | Sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preißelbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn maize |

Zur Verbreiterung des Wirkungsspektrums und zur Erzielung synergistischer Effekte können die neuen Dibenzofuranyloxyalkancarbonsäureester sowohl unter sich als auch mit zahlreichen Vertretern anderer herbizider oder wachstumsregulierender Wirkstoffgruppen gemischt und gemeinsam ausgebracht werden.

Sie werden beispielhaft aufgeführt:

5-Amino-4-chlor-2-phenyl-3(2H)-pyridazinon
5-Amino-4-brom-2-phenyl-3(2H)-pyridazinon
5-Amino-4-chlor-2-cyclohexyl-3(2H)-pyridazinon
5-Amino-4-brom-2-cyclohexyl-3(2H)-pyridazinon

5-Methylamino-4-chlor-2-(3.trifluormethylphenyl-3(2H)-pyridazinon
5-Methylamino-4-chlor-2-(3.α,α,ß,ß-tetrafluorethoxyphenyl)-3(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-cyclohexyl-3(2H)-pyridazinon
4,5-Dimethoxy-2-(3.trifluormethylphenyl)-3(2H)-pyridazinon
5-Methoxy-4-chlor-2-(3.trifluormethylphenyl)-3(2H)-pyridazinon
5-Amino-4-brom-2-(3.methylphenyl)-3(2H)-pyridazinon

3-(1-Methylethyl)-1H-2,1,3-benzothiadiazin-4(3H)-on-2,2-dioxid und Salze
3-(1-Methylethyl)-8-chlor-1H-2,1,3-benzothiadiazin-4(3H)-on-2,2-dioxid und Salze
3-(1-Methylethyl)-8-fluor-1H-2,1,3-benzothiadiazin-4(3H)-on-2,2-dioxid und Salze
3-(1-Methylethyl)-8-methyl-1H-2,1,3-benzothiadiazin-4(3H)-on-2,2-dioxid und Salze

1-Methoxymethyl-3-(1-methylethyl-2,1,3-benzothiadiazin-4(3H)-on-2,2-dioxid

1-Methoxymethyl-8-chlor-3-(1-methylethyl)-2,1,3-benzothiadiazin-4(3H)-on-2,2-dioxid

1-Methoxymethyl-8-fluor-3-(1-methylethyl)-2,1,3-benzothiadaizin-4(3H)-on-2,2-dioxid

1-Cyan-8-chlor-3-(1-methylethyl)-2,1,3-benzothiadiazin-4(3H)-on-2,2-dioxid

1-Cyan-8-fluor-3-(1-methylethyl)-2,1,3-benzothiadiazin-4(3H)-on-2,2-dioxid

1-Cyan-8-methyl-3-(1-methylethyl)-2,1,3-benzothiadiazin-4(3H)-on-2,2-dioxid

1-Cyan-3-(1-methylethyl)-2,1,3-benzothiadiazin-4(3H)-on-2,2-dioxid

1-Azidomethyl-3-(1-methylethyl)-2,1,3-benzothiadiazin-4(3H)-on-2,2-dioxid

3-(1-Methylethyl)-1H-pyridino-[3,2-e]2,1,3-thiadiazin-(4)-on-2,2-dioxid

N-(1-Ethylpropyl)-2,6-dinitro-3,4-dimethylanilin

N-(1-Methyläthyl)-N-ethyl-2,6-dinitro-4-trifluormethyl-anilin

N-n.Propyl-N-ß-chlorethyl-2,6-dinitro-4-trifluormethyl-anilin

N-n.Propyl-N-cyclopropylmethyl-2,6-dinitro-4-trifluormethyl-anilin

N-Bis-(n.propyl)-2,6-dinitro-3-amino-4-trifluormethyl-anilin

N-Bis-(n.propyl)-2,6-dinitro-4-methyl-anilin

N-Bis-(n.propyl)-2,6-dinitro-4-methylsulfonyl-anilin

N-Bis-(n.propyl)-2,6-dinitro-4-aminosulfonyl-anilin

Bis-(ß-chlorethyl)-2,6-dinitro-4-methyl-anilin

N-Ethyl-N-(2-methylallyl)-2,6-dinitro-4-trifluormethyl-anilin

N-Methylcarbaminsäure-3,4-dichlorbenzylester
N-Methylcarbaminsäure-2,6-di-tert.butyl-4-methylphenylester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
3'-N-Isopropyl-carbamoyloxy-propionsäureanilid

Ethyl-N-(3-(N'-phenylcarbamoyloxy)-phenyl)-carbamat
Methyl-N-(3-(N'-methyl-N'-phenylcarbamoyloxy)-phenyl)-carbamat
Isopropyl-N-(3-(N'-ethyl-N'-phenylcarbamoyloxy)-phenyl)-carbamat
Methyl-N-(3-(N'-3-methylphenylcarbamoyloxy)-phenyl)-carbamat
Methyl-N-(3-(N'-4-fluorphenylcarbamoyloxy)-phenyl)-carbamat
Methyl-N-(3-(N'-3-chlor-4-fluorphenylcarbamoyloxy)-phenyl)-carbamat
Ethyl-N-(3-(N'-3-chlor-4-fluorphenylcarbamoyloxy)-phenyl)-carbamat
Ethyl-N-(3-(N'-3,4-difluorphenylcarbamoyloxy)-phenyl)-carbamat
Methyl-N-(3-(N'-3,4-difluorphenylcarbamoyloxy)-phenyl)-carbamat

N-3-(4-Fluorphenoxycarbonylamino)-phenylcarbaminsäure-methylester
N-3-(2-Methylphenoxycarbonylamino)-phenylcarbaminsäure-ethylester

N-3-(4-Fluorphenoxycarbonylamino)-phenylthiolcarbaminsäure-methylester
N-3-(2,4,5-Trimethylphenoxycarbonylamino)-phenylthiolcarbaminsäure-methylester
N-3-(Phenoxycarbonylamino)-phenylthiolcarbaminsäuremethylester

N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolylmethylester
N,N-Di-isopropyl-thiolcarbaminsäure-3-ethyl-5-isoxazolylmethylester
N,N-Di-sec.butyl-thiolcarbaminsäure-ethylester
N,N-Di-sec.butyl-thiolcarbaminsäure-benzylester
N-Ethyl-N-cyclohexyl-thiolcarbaminsäure-ethylester
N-Ethyl-N-bicyclo-[2,2,1]-heptyl-thiolcarbaminsäure-ethylester
S-(2,3-Dichlorallyl)-(2,2,4-trimethyl-azetidin)-1-carbothiolat
S-(2,3,3-Trichlorallyl)-(2,2,4-trimethyl-azetidin)-1-carbothiolat
S-Ethyl-hexahydro-1-H-azepin-1-carbothiolat
S-Benzyl-3-methylhexahydro-1-H-azepin-1-carbothiolat
S-Benzyl-2,3-dimethylhexahydro-1-H-azepin-1-carbothiolat
S-Ethyl-3-methylhexahydro-1-H-azepin-1-carbothiolat
N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz

α,α-Dichlorpropionsäure-Natriumsalz
α,α-Dichlorbuttersäure-Natriumsalz
α,α,ß,ß-Tetrafluorpropionsäure-Natriumsalz
α-Methyl,α,ß-dichlorpropionsäure-Natriumsalz
α-Chlor-ß-(4-chlorphenyl)-propionsäure-methylester
α,ß-Dichlor-ß-phenylpropionsäure-methylester
Benzamido-oxy-essigsäure
2,3,5-Triiodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäuremethylester
2-[4-(2'-Chlor-4'-trifluorphenoxy)-phenoxy]-propionsäure-Natriumsalz
2-[4-(3',5'-Dichlorpyridyl-2-oxy)-phenoxy]-propionsäure-Natriumsalz

2-(N-Benzoyl-3,4-dichlorphenylamino)-propionsäureethylester
2-(N-Benzoyl-3-chlor-4-fluorphenylamino)-propionsäure-methylester
2-(N-Benzoyl-3-chlor-4-fluorphenylamino)-propionsäure-isopropylester

2-Chlor-4-ethylamino-6-isopropylamino-1,3,5-triazin
2-Chlor-4-ethylamino-6-(amino-2'-propionitril)-1,3,5-triazin
2-Chlor-4-ethylamino-6-2-methoxypropyl-2-amino-1,3,5-triazin
2-Chlor-4-ethylamino-6-butin-1-yl-2-amino-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin

2-Azido-4-methylamino-6-isopropylamino-1,3,5-triazin
2-Methylthio-4-ethylamino-6-isopropylamino-1,3,5-triazin
2-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin

2-Methoxy-4-ethylamino-6-isopropylamino-1,3,5-triazin
2-Methoxy-4,6-bisethylamino-1,3,5-triazin
2-Methoxy-4,6-bisiisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion

3-tert.Butyl-5-chlor-6-methyluracil
3-tert.Butyl-5-brom-6-methyluracil
3-Isopropyl-5-brom-6-methyluracil
3-sec.Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethyluracil

2-Methyl-4-(3'-trifluormethylphenyl)-tetrahydro-1,2,4-oxadiazin-3,5-dion

2-Methyl-4-(4'-fluorphenyl)-tetrahydro-1,2,4-oxadiazin-3,5-dion

3-Amino-1,2,4-triazol

1-Allyloxy-1-(4-bromphenyl)-2-[1',2',4'-triazolyl-(1')-]-ethan (Salze)

1-(4-Chlorphenoxy-3,3-dimethyl-1(1H-1,2,4-triazol-1-yl)-2-butanon

N,N-Diallylchloracetamid

N-Isopropyl-2-chloracetanilid

N-(1-Methyl-propin-2-yl)-2-chloracetanilid

2-Methyl-6-ethyl-N-(propargyl)-2-chloracetanilid

2-Methyl-6-ethyl-N-(ethoxymethyl)-2-chloracetanilid

2-Methyl-6-ethyl-N-(2-methoxy-1-methylethyl)-2-chloracetanilid

2-Methyl-6-ethyl-N-(isopropoxycarbonylethyl)-2-chloracetanilid

2-Methyl-6-ethyl-N-(4-methoxypyrazol-1-yl-methyl)-2-chloracetanilid

2-Methyl-6-ethyl-N-(pyrazol-1-yl-methyl)-2-chloracetanilid

2,6-Dimethyl-N-(pyrazol-1-yl-methyl)-2-chloracetanilid

2,6-Dimethyl-N-(4-methylpyrazol-1-yl-methyl)-2-chloracetanilid

2,6-Dimethyl-N-(1,2,4-triazol-1-yl-methyl)-2-chloracetanilid

2,6-Dimethyl-N-(3,5-dimethylpyrazol-1-yl-methyl)-2-chloracetanilid

2,6-Dimethyl-N-(1,3-dioxolan-2-yl-methyl)-2-chloracetanilid

2,6-Dimethyl-N-(2-methoxyethyl)-2-chloracetanilid
2,6-Dimethyl-N-(isobutoxymethyl)-2-chloracetanilid
2,6-Diethyl-N-(methoxymethyl)-2-chloracetanilid
2,6-Diethyl-N-(n-butoxymethyl)-2-chloracetanilid
2,6-Diethyl-N-(ethoxycarbonylmethyl)-2-chloracetanilid
2,3,6-Trimethyl-N-(pyrazol-1-yl-methyl)-2-chloracetanilid
2,3-Dimethyl-N-(isopropyl)-2-chloracetanilid
2,6-Diethyl-N-(2-n-propoxyethyl)-2-chloracetanilid

2-(2-Methyl-4-chlorphenoxy-N-methoxy-acetamid
2-(alpha-Naphtoxy)-N,N-diethylpropionamid
2,2-Diphenyl-N,N-dimethylacetamid
α-(3,4,5-Tribrompyrazol-1-yl)-N,N-dimethylpropionamid
N-(1,1-Dimethylpropinyl)-3,5-dichlorbenzamid
N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyltrifluormethan-sulfonanilid
5-Acetamido-4-methyl-trifluormethan-sulfonanilid

2-Propionyl-amino-4-methyl-5-chlor-thiazol
O-(Methylsulfonyl)-glykolsäure-N-ethoxymethyl-2,6-dimethylanilid
O-(Methylaminosulfonyl)-glykolsäure-N-isopropyl-anilid
O-(i-Propylaminosulfonyl)-glykolsäure-N-butin-1-yl-3--anilid
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Diiod-4-hydroxy-benzonitril (Salze)
3,5-Dibrom-4-hydroxy-O-2,4-dinitrophenylbenzaldoxim (Salze)
3,5-Dibrom-4-hydroxy-O-2-cyan-4-nitrophenylbenzaldoxim (Salze)

Pentachlorphenol-Natriumsalz
2,4-Dichlorphenyl-4'-nitrophenylether
2,4,6-Trichlorphenyl-4'-nitrophenylether
2-Fluor-4,6-dichlorphenyl-4'-nitrophenylether
2-Chlor-4-trifluormethylphenyl-4'-nitrophenylether
2,4'-Dinitro-4-trifluormethyl-diphenylether
2,4-Dichlorphenyl-3'-methoxy-4'-nitro-phenylether
2-Chlor-4-trifluormethylphenyl-3'-ethoxy-4'-nitro-phenylether
2-Chlor-4-trifluormethylphenyl-3'-carboxy-4'-nitro-phenylether (Salze)
2,4-Dichlorphenyl-3'-methoxycarbonyl-4'-nitro-phenylether
2-(3,4-Dichlorphenyl)-4-methyl-1,2,4-oxadiazolidin-3,5-dion
2-(3-tert.Butylcarbamoyl-oxyphenyl)-4-methyl-1,2,4-oxadiazolidin-3,5-dion
2-(3-iso-Propylcarbamoyl-oxyphenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$,$0^{8,11}$]-dodeca-3,9-dien
2-Ethoxy-2,3-dihydro-3,3-dimethyl-5-benzofuranyl-methansulfonat
2-Ethoxy-2,3-dihydro-3,3-dimethyl-5-benzofuranyl-dimethylaminosulfonat
2-Ethoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl-allylbernsteinsäureamid
2-Methyl-4,6-dinitrophenol (Salze, Ester)
2-sec.Butyl-4,6-dinitrophenol (Salze)
2-sec.Butyl-4,6-dinitrophenol-acetat
2-tert.Butyl-4,6-dinitrophenol-acetat

2-tert.Butyl-4,6-dinitrophenol (Salze)
2-tert.Butyl-5-methyl-4,6-dinitrophenol (Salze)

2-tert.Butyl-5-methyl-4,6-dinitrophenol-acetat

2-sec.Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-Chlorphenyl)-1-benzoyl-3,3-dimethyl-harnstoff
1-(4-Chlorphenyl)-3,3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(3-α,α,ß,ß-Tetrafluorethoxyphenyl)-3,3-dimethyl-harnstoff

1-(3-tert.-Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4(4'-Chlorphenoxy)-phenyl]-3,3-dimethyl-harnstoff
1-[4(4'-methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff

1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethylharnstoff
Imidazolidin-2-on-1-carbonsäure-iso-butylamid
1,2-Dimethyl-3,5-diphenylpyrazolium-methylsulfat
1,2,4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[(4-methylphenyl)-sulfonyl-oxy]-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1'-Dimethyl-4,4'-dipyridylium-di-(methylsulfat)
1,1'-Di(3,5-dimethylmorpholin-carbonylmethyl)-4,4'-dipyridylium-dichlorid
1,1'-Ethylen-2,2'-dipyridylium-dibromid
3-[1(N-Ethoxyamino)-propyliden]-6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino)-butyliden]-5,5-dimethylcyclohexan-1,3-dion (Salze)
2-[1-(N-Allyloxyamino)-butyliden]-5,5-dimethyl-4-methoxycarbonyl-cyclohexan-1,3-dion (Salze)
2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)

2,4,5-Trichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)
α-Naphthoxyessigsäuremethylester
2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy)-acrylat
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
Gibellerinsäure (Salze)
Dinatrium-methylarsonat
Mononatriumsalz der Methylarsonsäure
N-Phosphon-methyl-glycin (Salze)
N,N-Bis-(phosphonmethyl)-glycin (Salze)
2-Chlorethanphosphonsäure-2-chlorethylester
Ammonium-ethyl-carbamoyl-phosphonat
Di-n-butyl-1-n-butylamino-cyclohexyl-phosphonat
Trithiobutylphosphit
O,O-Diisopropyl-5-(2-benzosulfonylamino-ethyl)-phosphordithioat
2,3-Dihydro-5,6-dimethyl-1,4-dithiin-1,1,4,4-tetraoxid
5-tert.Butyl-3-(2,4-dichlor-5-isopropoxyphenyl)-1,3,4-oxadiazolon-(2)
4,5-Dichlor-2-trifluormethyl-benzimidazol (Salze)

1,2,3,6-Tetrahydropyridazin-3,6-dion (Salze)
Bernsteinsäure-mono-N-dimethylhydrazid (Salze)
(2-Chlorethyl)-trimethyl-ammoniumchlorid
(2-Methyl-4-phenylsulfonyl)-trifluormethansulfonanilid
1,1-Dimethyl-4,6-diisopropyl-5-indanylethylketon
Natriumchlorat
Ammoniumrhodanid
Calciumcyanamid
2-Chlor-4-trifluormethyl-3'-ethoxycarbonyl-4'-nitrophenylether

1-(4-Benzyloxyphenyl)-3-methyl-3-methoxyharnstoff
2-[1-(2,5-Dimethylphenyl)-ethylsulfonyl]-pyridin-N-oxid
1-Acetyl-3-anilino-4-methoxycarbonyl-5-methylpyrazol
3-Anilino-4-methoxycarbonyl-5-methylpyrazol
3-tert.Butylamino-4-methoxycarbonyl-5-methylpyrazol
N-Benzyl-N-isopropyl-trimethylacetamid
2-[4-(4'-Chlorphenoxymethyl)-phenoxy]-propionsäuremethylester
2-[4-(5'-Brompyridyl-2-oxy)-phenoxy]-propionsäureethylester
2-[4-(5'-Jodpyridyl-2-oxy)-phenoxy]-propionsäureethylester
2-[4-(5'-Jodpyridyl-2-oxy)-phenoxy]-propionsäure-n.-butylester
2-Chlor-4-trifluormethylphenyl-3'-(2-fluorethoxy)-4'-nitro-phenylether
2-Chlor-4-trifluormethylphenyl-3-(ethoxycarbonyl)methylthio-4-nitrophenylether
2,4,6-Trichlorphenyl-3(ethoxycarbonyl)methylthio-4-nitro-phenylether
2-[1-(N-ethoxamino)-butyliden]-5-(2-ethylthiopropyl)-3-hydroxy-cyclohexen-(2)-on-(1) (Salze)
2-[1-(N-ethoxamino)-butyliden]-5-(2-phenylthiopropyl)-3-hydroxy-cyclohexen-(2)-on-(1) (Salze)

4-[4-(4'-Trifluormethyl)-phenoxy]-penten-2-carbonsäure-ethylester
2-Chlor-4-trifluormethyl-3'-methoxycarbonyl-4'-nitrophenylether
2,4-Dichlorphenyl-3'-carboxy-4'-nitrophenylether (Salze)
4,5-Dimethoxy-2-(3-α,α,ß-trifluor-ß-bromethoxyphenyl)-3-(2H)-pyridazinon
2,4-Dichlorphenyl-3'-ethoxy-ethoxy-ethoxy-4'-nitrophenylether

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Zur Aktivierung der herbiziden Wirkung können auch Netz- und Haftmittel sowie nicht phytotoxische Öle und Ölkonzentrate zugesetzt werden.

Das Ergebnis eines Versuches zeigt, daß bei Anwendung im Gewächshaus im Nachauflaufverfahren die Wirkstoffe 3, 17, 25, 32, 33, 35 bei Aufwandmengen von 0,5 : 1 und 2 kg Wirkstoff je ha eine bessere herbizide Brauchbarkeit zeigen als der bekannte 2-(7-Chlor-3-dibenzofuranoxy)-propionsäure-tertiär-butylester oder das bekannte Dimethylammoniumsalz der 2-(2-Methyl-4-chlorphenoxy)-propionsäure.

Ein weiterer Versuch zeigt, daß bei Vorauflaufanwendung und bei Nachauflaufanwendung im Gewächshaus bei Aufwandmengen von 3 kg Wirkstoff je ha, die Wirkstoffe 6, 8, 24, 30, 23, 33, 44, 45, 46, 47, 48 und 49 eine gute herbizide Wirkung zeigen.

In einem weiteren Versuch bei Nachauflaufanwendung im Gewächshaus und Aufwandmengen von 0,25; 1,0 und 2,0 kg Wirkstoff je ha zeigten die Wirkstoffe 5, 38, 39, 41, 43, 1, 14, 12, 20, 22, 16, 28, 17, 18, 19, 15, 31, 2, 9, 21, 13, 36, 34 und 42 eine gute herbizide Wirkung.

Patentansprüche

1. Dibenzofuranoxyalkancarbonsäureester-Derivate der allgemeinen Formel

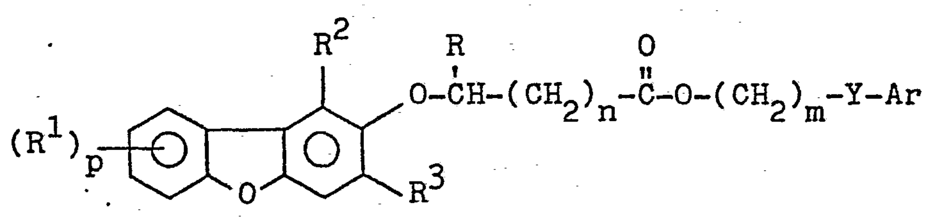

in welcher

$R^1$ Halogen, eine gegebenenfalls halogensubstituierte Alkyl- oder Alkoxygruppe mit 1 bis 4 C-Atomen, Cyan oder Nitro bedeutet,

$R^2$ und $R^3$ gleich oder verschieden sind und unabhängig voneinander Wasserstoff, Halogen oder Nitro bedeuten,

R Wasserstoff, Methyl oder Ethyl,

m die Zahlen 2, 3, 4, 5 oder 6

n die Zahlen 0 oder 2

p die Zahlen 1 oder 2 und

Y ein Sauerstoff- oder Schwefelatom bedeuten und

Ar 3-Dibenzofuranyl, 1,2- oder 1,4-Biphenyl, 1- oder 2-Naphthyl oder Phenyl bedeuten, wobei der Phenylrest durch Halogen, durch gegebenenfalls halogensubstituierte Alkyl-, Alkenyl-, Alkoxy- oder Alkylthiogruppen mit 1 bis 5 C-Atomen oder durch Acetyl, Cyan oder Nitro substituiert sein kann.

2. Dibenzofuranoxyalkancarbonsäureester gemäß Anspruch 1 nämlich 2-(7-Fluor-3-dibenzofuranoxy)-propionsäure-2'-(3-methylphenoxy)-ethylester; 2-(7-Chlor-3-dibenzofuranoxy)-propionsäure-2'-phenoxyethylester; 2-(7-Chlor-3-dibenzofuranoxy)-propionsäure-3'-phenoxypropylester; 2-(7-Chlor-3-dibenzofuranoxy)-propionsäure-4'-phenoxybutylester; 2-(7-Chlor-3-dibenzofuranoxy)-propionsäure-2'-(3-chlorphenoxy)-ethylester; 2-(7-Chlor-3-dibenzofuranoxy)-propionsäure-2'-(4-chlorphenylthio)-ethylester; 2-(7-Chlor-3-dibenzofuranoxy)-propionsäure-2'-(3-methylphenoxy)-ethylester; 2-(7-Chlor-3-dibenzofuranoxy)-propionsäure-2'-(3-trifluormethylphenoxy)-ethylester; 2-(7-Chlor-3-dibenzofuranoxy)-propionsäure-2'-(1-naphthyloxy)-ethylester; 2-(7-Chlor-3-dibenzofuranoxy)-propionsäure-2'-(3,5-dichlorphenoxy)-ethylester; 2-(7-Chlor-3-dibenzofuranoxy)-propionsäure-2'-(2,4-dimethylphenoxy)-ethylester; 2-(7-Brom-3-dibenzofuranoxy)-propionsäure-2'-phenoxyethylester; 2-(7-Brom-3-dibenzofuranoxy)-propionsäure-2'-(3-chlorphenoxy)-ethylester; 2-(7-Brom-3-dibenzofuranoxy)-propionsäure-2'-(4-chlorphenylthio)-ethylester; 2-(7-Brom-3-dibenzofuranoxy)-propionsäure-2'-(3-methylphenoxy)-ethylester; 2-(7-Brom-3-dibenzofuranoxy)-propionsäure-2'-(3-trifluormethylphenoxy)-ethylester; 2-(7-Brom-3-dibenzofuranoxy)-propionsäure-4'-(3-trifluormethylphenoxy)-butylester; 2-(7-Tetrafluorethoxy-3-dibenzofuranoxy)-propionsäure-2'-(3-methylphenoxy)-ethylester; 2-(7-Trifluormethyl-3-dibenzofuranoxy)-propionsäure-2'-phenoxyethylester und 2-(7-Trifluormethyl-3-dibenzofuranoxy)-propionsäure-2'-(3-chlorphenoxy)-ethylester.

3. Herbizid, enthaltend einen Dibenzofuranoxyalkancarbonsäureester der allgemeinen Formel

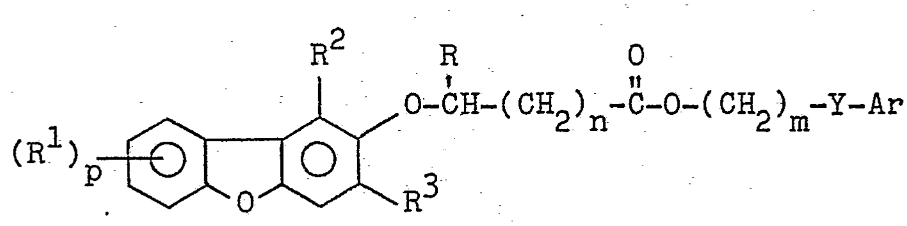

in welcher

$R^1$ Halogen, eine gegebenenfalls halogensubstituierte Alkyl- oder Alkoxygruppe mit 1 bis 4 C-Atomen, Cyan oder Nitro bedeutet,

$R^2$ und $R^3$ gleich oder verschieden sind und unabhängig voneinander Wasserstoff, Halogen oder Nitro bedeuten,

R Wasserstoff, Methyl oder Ethyl,

m die Zahlen 2, 3, 4, 5 oder 6

n die Zahlen 0 oder 2

p die Zahlen 1 oder 2 und

Y ein Sauerstoff- oder Schwefelatom bedeuten und

Ar 3-Dibenzofuranyl, 1,2- oder 1,4-Biphenyl, 1- oder 2-Naphthyl oder Phenyl bedeuten, wobei der Phenylrest durch Halogen, durch gegebenenfalls halogensubstituierte Alkyl-, Alkenyl-, Alkoxy- oder Alkylthiogruppen mit 1 bis 5 C-Atomen oder durch Acetyl, Cyan oder Nitro substituiert sein kann.

4. Herbizid, enthaltend einen Dibenzofuranoxyalkancarbonsäureester der allgemeinen Formel

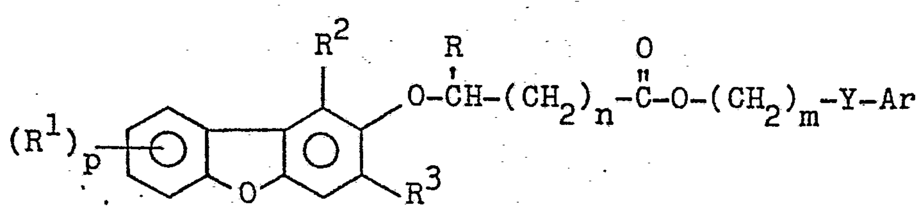

in welcher

$R^1$ Halogen, eine gegebenenfalls halogensubstituierte Alkyl- oder Alkoxygruppe mit 1 bis 4 C-Atomen, Cyan oder Nitro bedeutet,

$R^2$ und $R^3$ gleich oder verschieden sind und unabhängig voneinander Wasserstoff, Halogen oder Nitro bedeuten,

R Wasserstoff, Methyl oder Ethyl,

m die Zahlen 2, 3, 4, 5 oder 6

n die Zahlen 0 oder 2

p die Zahlen 1 oder 2 und

Y ein Sauerstoff- oder Schwefelatom bedeuten und

Ar 3-Dibenzofuranyl, 1,2- oder 1,4-Biphenyl, 1- oder 2-Naphthyl oder Phenyl bedeuten, wobei der Phenylrest durch Halogen, durch gegebenenfalls halogensubstituierte Alkyl-, Alkenyl-, Alkoxy- oder Alkylthiogruppen mit 1 bis 5 C-Atomen oder durch Acetyl, Cyan oder Nitro substituiert sein kann.

5. Verfahren zur Herstellung eines Herbizids, dadurch gekennzeichnet, daß man einen festen oder flüssigen Trägerstoff vermischt mit einem Dibenzofuranoxyalkancarbonsäureester der allgemeinen Formel

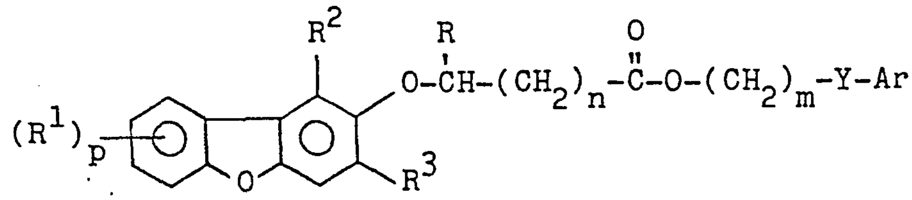

in welcher

$R^1$ Halogen, eine gegebenenfalls halogensubstituierte Alkyl- oder Alkoxygruppe mit 1 bis 4 C-Atomen, Cyan oder Nitro bedeutet,

$R^2$ und $R^3$ gleich oder verschieden sind und unabhängig voneinander Wasserstoff, Halogen oder Nitro bedeuten,

R Wasserstoff, Methyl oder Ethyl,

m die Zahlen 2, 3, 4, 5 oder 6

n die Zahlen 0 oder 2

p die Zahlen 1 oder 2 und

Y ein Sauerstoff- oder Schwefelatom bedeuten und

Ar 3-Dibenzofuranyl, 1,2- oder 1,4-Biphenyl, 1- oder 2-Naphthyl oder Phenyl bedeuten, wobei der Phenylrest durch Halogen, durch gegebenenfalls halogensubstituierte Alkyl-, Alkenyl-, Alkoxy- oder Alkylthiogruppen mit 1 bis 5 C-Atomen oder durch Acetyl, Cyan oder Nitro substituiert sein kann.

6. Verfahren zur Bekämpfung unerwünschten Pflanzenwuchses, dadurch gekennzeichnet, daß man die Pflanzen oder den Boden behandelt mit einem Dibenzofuranoxyalkancarbonsäureester der allgemeinen Formel

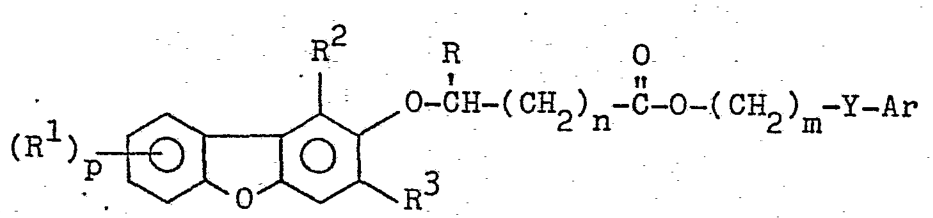

in welcher

$R^1$ Halogen, eine gegebenenfalls halogensubstituierte Alkyl- oder Alkoxygruppe mit 1 bis 4 C-Atomen, Cyan oder Nitro bedeutet,

$R^2$ und $R^3$ gleich oder verschieden sind und unabhängig voneinander Wasserstoff, Halogen oder Nitro bedeuten,

R Wasserstoff, Methyl oder Ethyl,

m die Zahlen 2, 3, 4, 5 oder 6

n die Zahlen 0 oder 2

p die Zahlen 1 oder 2 und

Y ein Sauerstoff- oder Schwefelatom bedeuten und

Ar 3-Dibenzofuranyl, 1,2- oder 1,4-Biphenyl, 1- oder 2-Naphthyl oder Phenyl bedeuten, wobei der Phenylrest durch Halogen, durch gegebenenfalls halogensubstituierte Alkyl-, Alkenyl-, Alkoxy- oder Alkylthiogruppen mit 1 bis 5 C-Atomen oder durch Acetyl, Cyan oder Nitro substituiert sein kann.

7. Herbizid, enthaltend einen Dibenzofuranoxyalkancarbonsäureester nämlich 2-(7-Fluor-3-dibenzofuranoxy)-propionsäure-2'-(3--methylphenoxy)-ethylester; 2-(7-Chlor-3-dibenzofuranoxy)-propionsäure-2'-phenoxyethylester; 2-(7-Chlor-3--dibenzofuranoxy)-propionsäure-3'-phenoxypropylester; 2-(7-Chlor-3-dibenzofuranoxy)-propionsäure-4'-phenoxybutylester; 2-(7-Chlor-3-dibenzofuranoxy)-propionsäure-2'-(3-chlorphenoxy)-ethylester; 2-(7-Chlor-3--dibenzofuranoxy)-propionsäure-2'-(4-chlorphenylthio)-ethylester; 2-(7-Chlor-3-dibenzofuranoxy)-propionsäure-2'-(3-methylphenoxy)-ethylester; 2-(7-Chlor--3-dibenzofuranoxy)-propionsäure-2'-(3-trifluormethylphenoxy)-ethylester; 2-(7-Chlor-3-dibenzofuranoxy)--propionsäure-2'-(1-naphthyloxy)-ethylester; 2-(7-Chlor-3-dibenzofuranoxy)-propionsäure-2'-(3,5--dichlorphenoxy)-ethylester; 2-(7-Chlor-3-dibenzofuranoxy)-propionsäure-2'-(2,4-dimethylphenoxy)--ethylester; 2-(7-Brom-3-dibenzofuranoxy)-propionsäure-2'-phenoxyethylester; 2-(7-Brom-3-dibenzofuranoxy)-propionsäure-2'-(3-chlorphenoxy)-ethylester; 2-(7-Brom-3-dibenzofuranoxy)-propionsäure-2'-(4-chlorphenylthio)-ethylester; 2-(7-Brom-3-dibenzofuranoxy)-propionsäure-2'-(3-methylphenoxy)-ethylester; 2-(7-Brom-3-dibenzofuranoxy)-propionsäure-2'-(3-trifluormethylphenoxy)-ethylester; 2-(7-Brom-3-dibenzofuranoxy)-propionsäure-4'-(3-trifluormethylphenoxy)-butylester; 2-(7-Tetrafluorethoxy-3-dibenzofuranoxy)-propionsäure-2'-(3-methylphenoxy)-ethylester; 2-(7-Trifluormethyl-3-dibenzofuranoxy)-propionsäure-2'-phenoxyethylester und 2-(7-Trifluormethyl-3-dibenzofuranoxy)-propionsäure-2'-(3-chlorphenoxy)-ethylester.

8. Verfahren zur Herstellung eines Dibenzofuranoxyalkancarbonsäureesters der allgemeinen Formel

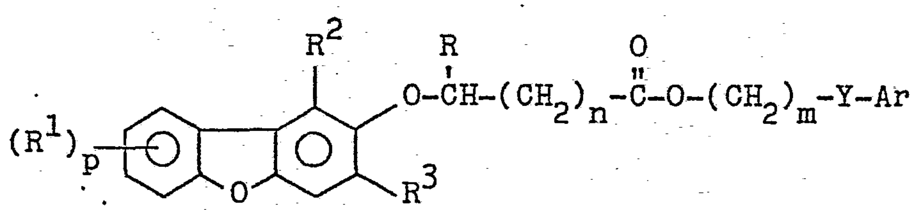

in welcher

$R^1$ Halogen, eine gegebenenfalls halogensubstituierte Alkyl- oder Alkoxygruppe mit 1 bis 4 C-Atomen, Cyan oder Nitro bedeutet,

$R^2$ und $R^3$ gleich oder verschieden sind und unabhängig voneinander Wasserstoff, Halogen oder Nitro bedeuten,

R Wasserstoff, Methyl oder Ethyl,

m die Zahlen 2, 3, 4, 5 oder 6

n die Zahlen 0 oder 2

p die Zahlen 1 oder 2 und

Y ein Sauerstoff- oder Schwefelatom bedeuten und

Ar 3-Dibenzofuranyl, 1,2- oder 1,4-Biphenyl, 1- oder 2-Naphthyl oder Phenyl bedeuten, wobei der Phenylrest durch Halogen, durch gegebenenfalls halogensubstituierte Alkyl-, Alkenyl-, Alkoxy- oder Alkylthiogruppen mit 1 bis 5 C-Atomen oder durch Acetyl, Cyan oder Nitro substituiert sein kann,

dadurch gekennzeichnet, daß man einen Ester der Formel

$$Z-\overset{R}{C}H-(CH_2)_n-\overset{O}{\overset{\|}{C}}-O-(CH_2)_m-Y-Ar$$

in welcher

Z ein Halogenatom bedeutet und

R, Y, Ar, m und n die in Anspruch 1 angegebenen Bedeutungen haben,

a) mit einem substituierten Dibenzofuranol der Formel

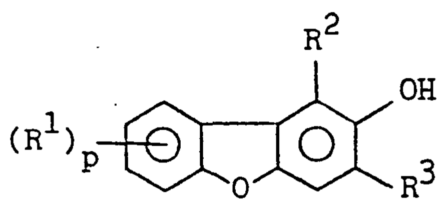

oder

b) mit dem Metallsalz eines substituierten Dibenzofuranols

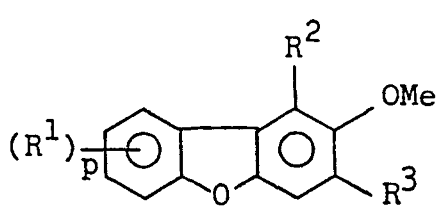

in welchen
Me Lithium, Natrium oder Kalium bedeutet und $R^1$, $R^2$, $R^3$ und p die in Anspruch 1 angegebenen Bedeutungen haben, gegebenenfalls in Gegenwart eines Lösungs- oder Verdünnungsmittels, gegebenenfalls unter Zusatz einer anorganischen Base und gegebenenfalls unter Zusatz eines Reakionsbeschleunigers, bei Temperaturen zwischen 20 und 150°C umsetzt.

9. Verfahren zur Herstellung eines Dibenzofuranoxyalkancarbonsäureesters der allgemeinen Formel

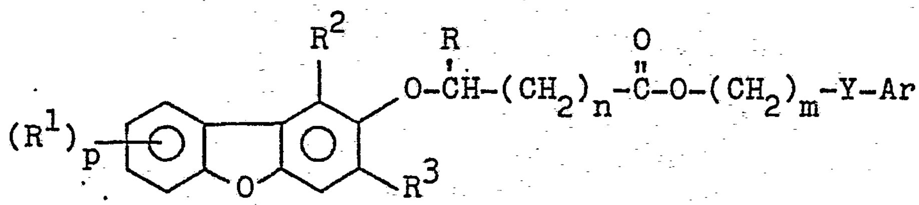

in welcher
$R^1$ Halogen, eine gegebenenfalls halogensubstituierte Alkyl- oder Alkoxygruppe mit 1 bis 4 C-Atomen, Cyan oder Nitro bedeutet,
$R^2$ und $R^3$ gleich oder verschieden sind und unabhängig voneinander Wasserstoff, Halogen oder Nitro bedeuten,
R Wasserstoff, Methyl oder Ethyl,
m die Zahlen 2, 3, 4, 5 oder 6
n die Zahlen 0 oder 2
p die Zahlen 1 oder 2 und
Y ein Sauerstoff- oder Schwefelatom bedeuten und
Ar 3-Dibenzofuranyl, 1,2- oder 1,4-Biphenyl, 1- oder 2-Naphthyl oder Phenyl bedeuten, wobei der Phenylrest durch Halogen, durch gegebenenfalls halogensubstituierte Alkyl-, Alkenyl-, Alkoxy- oder Alkylthiogruppen mit 1 bis 5 C-Atomen oder durch Acetyl, Cyan oder Nitro substituiert sein kann,

dadurch gekennzeichnet, daß man einen Alkohol der Formel

$$HO-(CH_2)_m-Y-Ar$$

in welcher
Ar, Y und m die in Anspruch 1 angegebenen Bedeutungen haben,

a) mit einer Dibenzofuranoxyalkancarbonsäure der Formel

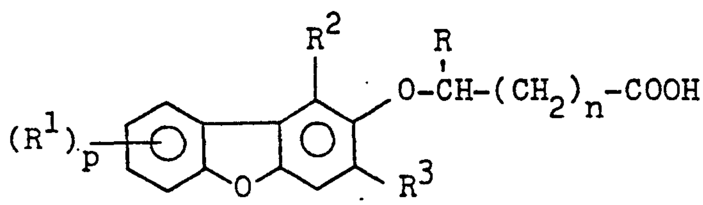

in welcher
$R^1$, $R^2$, $R^3$, n und p die in Anspruch 1 angegebenen Bedeutungen haben, gegebenenfalls in Gegenwart eines Lösungs- oder Verdünnungsmittels, gegebenenfalls unter Zusatz einer anorganischen, organischen oder polymeren starken Säure und gegebenenfalls unter Wasserabscheidung, bei Temperaturen zwischen 20 und 150°C verestert, oder

b) mit einem Dibenzofuranoxyalkancarbonsäure-halogenid der Formel

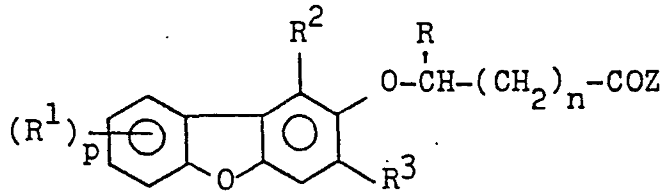

in welcher

Z ein Halogenatom bedeutet und $R^1$, $R^2$, $R^3$, R, n und p die in Anspruch 1 und 2 angegebenen Bedeutungen haben, gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers, bei Temperaturen von 0 bis 120°C umsetzt, oder

c) mit einem Dibenzofuranoxyalkancarbonsäureester der Formel

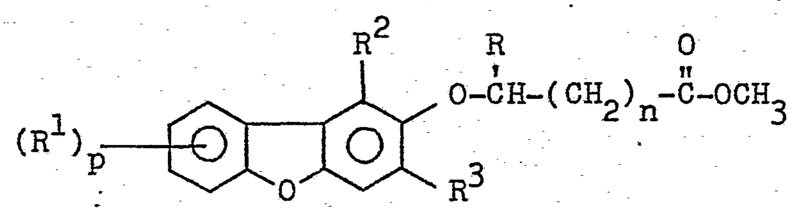

in welcher $R^1$, $R^2$, $R^3$, R, n und p die in Anspruch 1 angegebenen Bedeutungen haben, gegebenenfalls in Gegenwart eines Lösungs- oder Verdünnungsmittels, gegebenenfalls unter Zusatz einer starken anorganischen oder organischen Base oder einer starken anorganischen oder organischen Säure oder einer Lewis-Säure oder einem Metalloxid- oder -alkoholat bei Temperaturen von 0 bis 150°C umsetzt.

10. Verfahren zur Herstellung eines Dibenzofuranoxyalkancarbonsäureesters der allgemeinen Formel

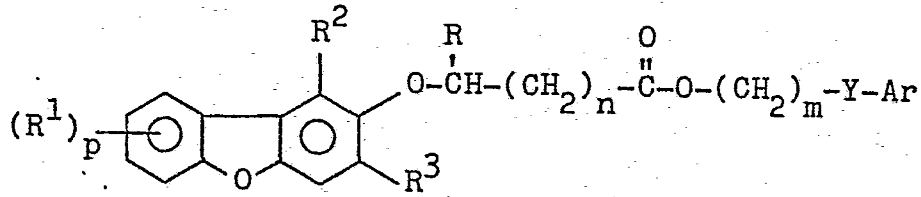

in welcher

$R^1$ Halogen, eine gegebenenfalls halogensubstituierte Alkyl- oder Alkoxygruppe mit 1 bis 4 C-Atomen, Cyan oder Nitro bedeutet,

$R^2$ und $R^3$ gleich oder verschieden sind und unabhängig voneinander Wasserstoff, Halogen oder Nitro bedeuten,

R Wasserstoff, Methyl oder Ethyl,

m die Zahlen 2, 3, 4, 5 oder 6

n die Zahlen 0 oder 2

p die Zahlen 1 oder 2 und

Y ein Sauerstoff- oder Schwefelatom bedeuten und

Ar 3-Dibenzofuranyl, 1,2- oder 1,4-Biphenyl, 1- oder 2-Naphthyl oder Phenyl bedeuten, wobei der Phenylrest durch Halogen, durch gegebenenfalls halogensubstituierte Alkyl-, Alkenyl-, Alkoxy- oder Alkylthiogruppen mit 1 bis 5 C-Atomen oder durch Acetyl, Cyan oder Nitro substituiert sein kann,

dadurch gekennzeichnet, daß man ein Dibenzofuranoxyalkancarbonsäure-Metallsalz der Formel

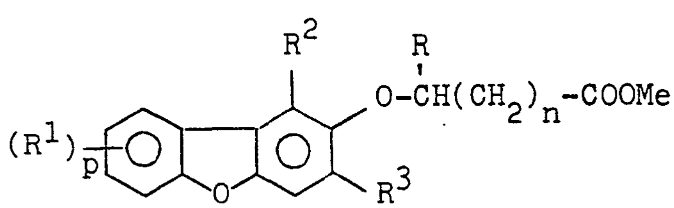

in welcher

$R^1$, $R^2$, $R^3$, R, Me, n und P die in den Ansprüchen 1 und 2 angegebenen Bedeutungen haben, mit einem Aryloxy- oder Arylthio-alkylhalogenid der Formel

$$Z-(CH_2)_m-Y-Ar$$

in welcher
Ar, Y, Z und m die in den Ansprüchen 1 und 2 angegebenen Bedeutungen haben, gegebenenfalls in Gegenwart eines Lösungs- oder Verdünnungsmittels und gegebenenfalls unter Zusatz eines Reaktionsbeschleunigers, bei Temperaturen zwischen 0 und 150°C umsetzt.

Patentansprüche (Österreich)

1. Herbizid, enthaltend einen Dibenzofuranoxyalkancarbonsäureester der allgemeinen Formel

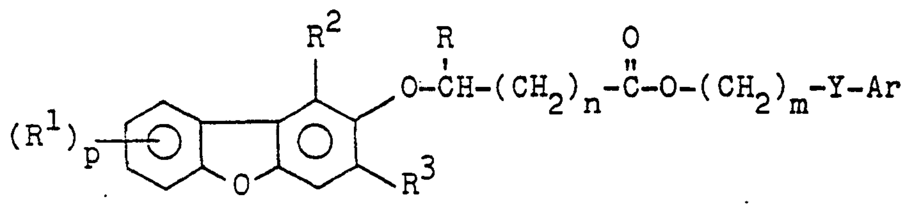

in welcher

$R^1$ Halogen, eine gegebenenfalls halogensubstituierte Alkyl- oder Alkoxygruppe mit 1 bis 4 C-Atomen, Cyan oder Nitro bedeutet,

$R^2$ und $R^3$ gleich oder verschieden sind und unabhängig voneinander Wasserstoff, Halogen oder Nitro bedeuten,

R Wasserstoff, Methyl oder Ethyl,

m die Zahlen 2, 3, 4, 5 oder 6

n die Zahlen 0 oder 2

p die Zahlen 1 oder 2 und

Y ein Sauerstoff- oder Schwefelatom bedeuten und

Ar 3-Dibenzofuranyl, 1,2- oder 1,4-Biphenyl, 1- oder 2-Naphthyl oder Phenyl bedeuten, wobei der Phenylrest durch Halogen, durch gegebenenfalls halogensubstituierte Alkyl-, Alkenyl-, Alkoxy- oder Alkylthiogruppen mit 1 bis 5 C-Atomen oder durch Acetyl, Cyan oder Nitro substituiert sein kann.

2. Herbizid, enthaltend einen Dibenzofuranoxyalkancarbonsäureester der allgemeinen Formel

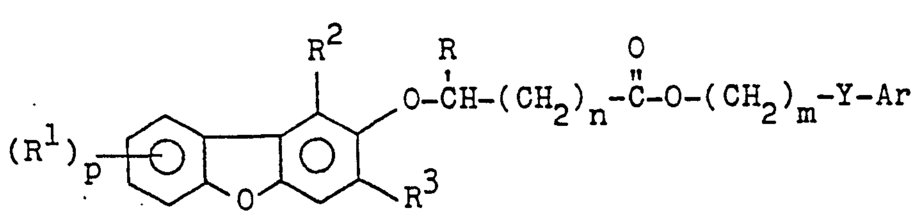

in welcher

$R^1$ Halogen, eine gegebenenfalls halogensubstituierte Alkyl- oder Alkoxygruppe mit 1 bis 4 C-Atomen, Cyan oder Nitro bedeutet,

$R^2$ und $R^3$ gleich oder verschieden sind und unabhängig voneinander Wasserstoff, Halogen oder Nitro bedeuten,

R Wasserstoff, Methyl oder Ethyl,

m die Zahlen 2, 3, 4, 5 oder 6

n die Zahlen 0 oder 2

p die Zahlen 1 oder 2 und

Y ein Sauerstoff- oder Schwefelatom bedeuten und

Ar 3-Dibenzofuranyl, 1,2- oder 1,4-Biphenyl, 1- oder 2-Naphthyl oder Phenyl bedeuten, wobei der Phenylrest durch Halogen, durch gegebenenfalls halogensubstituierte Alkyl-, Alkenyl-, Alkoxy- oder Alkylthiogruppen mit 1 bis 5 C-Atomen oder durch Acetyl, Cyan oder Nitro substituiert sein kann.

3. Verfahren zur Herstellung eines Herbizids, dadurch gekennzeichnet, daß man einen festen oder flüssigen Trägerstoff vermischt mit einem Dibenzofuranoxyalkancarbonsäureester der allgemeinen Formel

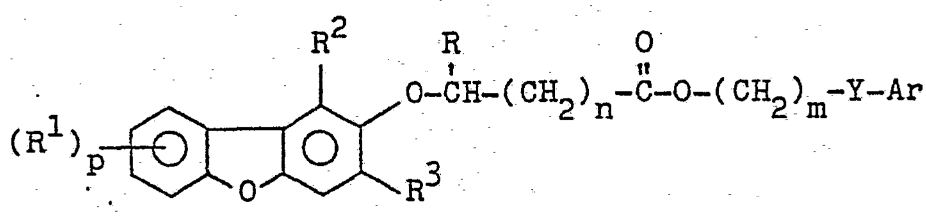

in welcher

$R^1$ Halogen, eine gegebenenfalls halogensubstituierte Alkyl- oder Alkoxygruppe mit 1 bis 4 C-Atomen, Cyan oder Nitro bedeutet,

$R^2$ und $R^3$ gleich oder verschieden sind und unabhängig voneinander Wasserstoff, Halogen oder Nitro bedeuten,

R Wasserstoff, Methyl oder Ethyl,

m die Zahlen 2, 3, 4, 5 oder 6

n die Zahlen 0 oder 2

p die Zahlen 1 oder 2 und

Y ein Sauerstoff- oder Schwefelatom bedeuten und

Ar 3-Dibenzofuranyl, 1,2- oder 1,4-Biphenyl, 1- oder 2-Naphthyl oder Phenyl bedeuten, wobei der Phenylrest durch Halogen, durch gegebenenfalls halogensubstituierte Alkyl-, Alkenyl-, Alkoxy- oder Alkylthiogruppen mit 1 bis 5 C-Atomen oder durch Acetyl, Cyan oder Nitro substituiert sein kann.

4. Verfahren zur Bekämpfung unerwünschten Pflanzenwuchses, dadurch gekennzeichnet, daß man die Pflanzen oder den Boden behandelt mit einem Dibenzofuranoxyalkancarbonsäureester der allgemeinen Formel

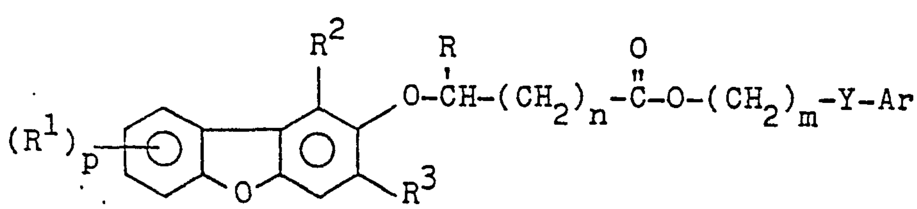

in welcher

$R^1$ Halogen, eine gegebenenfalls halogensubstituierte Alkyl- oder Alkoxygruppe mit 1 bis 4 C-Atomen, Cyan oder Nitro bedeutet,

| | |
|---|---|
| $R^2$ und $R^3$ | gleich oder verschieden sind und unabhängig voneinander Wasserstoff, Halogen oder Nitro bedeuten, |
| R | Wasserstoff, Methyl oder Ethyl, |
| m | die Zahlen 2, 3, 4, 5 oder 6 |
| n | die Zahlen 0 oder 2 |
| p | die Zahlen 1 oder 2 und |
| Y | ein Sauerstoff- oder Schwefelatom bedeuten und |
| Ar | 3-Dibenzofuranyl, 1,2- oder 1,4-Biphenyl, 1- oder 2-Naphthyl oder Phenyl bedeuten, wobei der Phenylrest durch Halogen, durch gegebenenfalls halogensubstituierte Alkyl-, Alkenyl-, Alkoxy- oder Alkylthiogruppen mit 1 bis 5 C-Atomen oder durch Acetyl, Cyan oder Nitro substituiert sein kann. |

5. Herbizid, enthaltend einen Dibenzofuranoxyalkancarbonsäureester nämlich 2-(7-Fluor-3-dibenzofuranoxy)-propionsäure-2'-(3-methylphenoxy)-ethylester; 2-(7-Chlor-3-dibenzofuranoxy)-propionsäure-2'-phenoxyethylester; 2-(7-Chlor-3-dibenzofuranoxy)-propionsäure-3'-phenoxypropylester; 2-(7-Chlor-3-dibenzofuranoxy)-propionsäure-4'-phenoxybutylester; 2-(7-Chlor-3-dibenzofuranoxy)-propionsäure-2'-(3-chlorphenoxy)-ethylester; 2-(7-Chlor-3-dibenzofuranoxy)-propionsäure-2'-(4-chlorphenylthio)-ethylester; 2-(7-Chlor-3-dibenzofuranoxy)-propionsäure-2'-(3-methylphenoxy)-ethylester; 2-(7-Chlor-3-dibenzofuranoxy)-propionsäure-2'-(3-trifluormethylphenoxy)-ethylester; 2-(7-Chlor-3-dibenzofuranoxy)-propionsäure-2'-(1-naphthyloxy)-ethylester; 2-(7-Chlor-3-dibenzofuranoxy)-propionsäure-2'-(3,5-dichlorphenoxy)-ethylester; 2-(7-Chlor-3-dibenzofuranoxy)-propionsäure-2'-(2,4-dimethylphenoxy)-ethylester; 2-(7-Brom-3-dibenzofuranoxy)-propionsäure-2'-phenoxyethylester; 2-(7-Brom-3-dibenzofuranoxy)-propionsäure-2'-(3-chlorphenoxy)-ethylester; 2-(7-Brom-3-dibenzofuranoxy)-propionsäure-2'-(4-chlorphenylthio)-ethylester; 2-(7-Brom-3-dibenzofuranoxy)-propionsäure-2'-(3-methylphenoxy)-ethylester; 2-(7-Brom-3-dibenzofuranoxy)-propionsäure-2'-(3-trifluormethylphenoxy)-ethylester; 2-(7-Brom-3-dibenzofuranoxy)-propionsäure-4'-(3-trifluormethylphenoxy)-butylester; 2-(7-Tetrafluorethoxy-3-dibenzofuranoxy)-propionsäure-2'-(3-methylphenoxy)-ethylester; 2-(7-Trifluormethyl-3-dibenzofuranoxy)-propionsäure-2'-phenoxyethylester und 2-(7-Trifluormethyl-3-dibenzofuranoxy)-propionsäure-2'-(3-chlorphenoxy)-ethylester.

6. Verfahren zur Herstellung eines Dibenzofuranoxyalkancarbonsäureesters der allgemeinen Formel

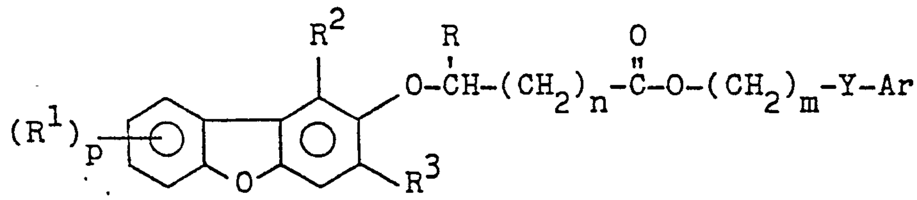

in welcher

$R^1$ Halogen, eine gegebenenfalls halogensubstituierte Alkyl- oder Alkoxygruppe mit 1 bis 4 C-Atomen, Cyan oder Nitro bedeutet,

$R^2$ und $R^3$ gleich oder verschieden sind und unabhängig voneinander Wasserstoff, Halogen oder Nitro bedeuten,

R Wasserstoff, Methyl oder Ethyl,

m die Zahlen 2, 3, 4, 5 oder 6

n die Zahlen 0 oder 2

p die Zahlen 1 oder 2 und

Y ein Sauerstoff- oder Schwefelatom bedeuten und

Ar 3-Dibenzofuranyl, 1,2- oder 1,4-Biphenyl, 1- oder 2-Naphthyl oder Phenyl bedeuten, wobei der Phenylrest durch Halogen, durch gegebenenfalls halogensubstituierte Alkyl-, Alkenyl-, Alkoxy- oder Alkylthiogruppen mit 1 bis 5 C-Atomen oder durch Acetyl, Cyan oder Nitro substituiert sein kann,

dadurch gekennzeichnet, daß man einen Ester der Formel

$$Z-\overset{R}{\overset{|}{C}}H-(CH_2)_n-\overset{O}{\overset{\|}{C}}-O-(CH_2)_m-Y-Ar$$

in welcher

Z ein Halogenatom bedeutet und

R, Y, Ar, m und n die in Anspruch 1 angegebenen Bedeutungen haben,

a) mit einem substituierten Dibenzofuranol der Formel

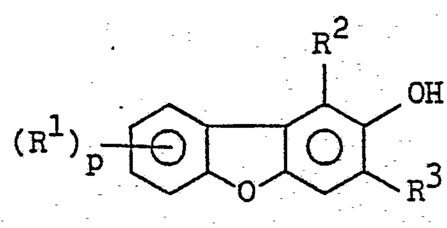

oder

b) mit dem Metallsalz eines substituierten Dibenzofuranols

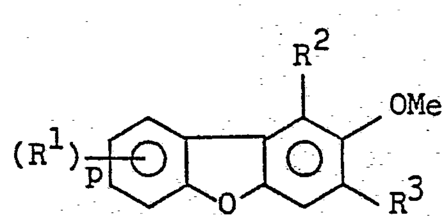

in welchen
Me Lithium, Natrium oder Kalium bedeutet und
$R^1$, $R^2$, $R^3$ und p die in Anspruch 1 angegebenen Bedeutungen haben, gegebenenfalls in Gegenwart eines Lösungs- oder Verdünnungsmittels, gegebenenfalls unter Zusatz einer anorganischen Base und gegebenenfalls unter Zusatz eines Reakionsbeschleunigers, bei Temperaturen zwischen 20 und 150°C umsetzt.

7. Verfahren zur Herstellung eines Dibenzofuranoxyalkancarbonsäureesters der allgemeinen Formel

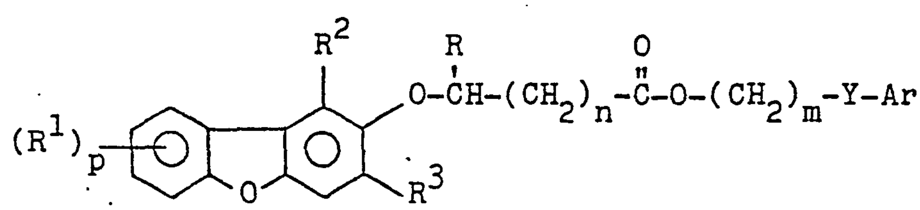

in welcher
$R^1$ Halogen, eine gegebenenfalls halogensubstituierte Alkyl- oder Alkoxygruppe mit 1 bis 4 C-Atomen, Cyan oder Nitro bedeutet,
$R^2$ und $R^3$ gleich oder verschieden sind und unabhängig voneinander Wasserstoff, Halogen oder Nitro bedeuten,
R Wasserstoff, Methyl oder Ethyl,
m die Zahlen 2, 3, 4, 5 oder 6
n die Zahlen 0 oder 2
p die Zahlen 1 oder 2 und
Y ein Sauerstoff- oder Schwefelatom bedeuten und
Ar 3-Dibenzofuranyl, 1,2- oder 1,4-Biphenyl, 1- oder 2-Naphthyl oder Phenyl bedeuten, wobei der Phenylrest durch Halogen, durch gegebenenfalls halogensubstituierte Alkyl-, Alkenyl-, Alkoxy- oder Alkylthiogruppen mit 1 bis 5 C-Atomen oder durch Acetyl, Cyan oder Nitro substituiert sein kann,

dadurch gekennzeichnet, daß man einen Alkohol der Formel

$$HO-(CH_2)_m-Y-Ar$$

in welcher
Ar, Y und m die in Anspruch 1 angegebenen Bedeutungen haben,

a) mit einer Dibenzofuranoxyalkancarbonsäure der Formel

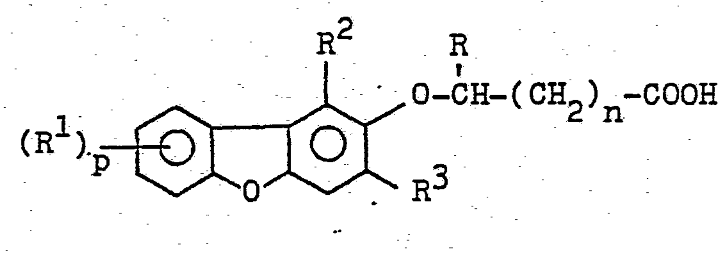

in welcher
$R^1$, $R^2$, $R^3$, n und p die in Anspruch 1 angegebenen Bedeutungen haben, gegebenenfalls in Gegenwart eines Lösungs- oder Verdünnungsmittels, gegebenenfalls unter Zusatz einer anorganischen, organischen oder polymeren starken Säure und gegebenenfalls unter Wasserabscheidung, bei Temperaturen zwischen 20 und 150°C verestert, oder

b) mit einem Dibenzofuranoxyalkancarbonsäure-halogenid der Formel

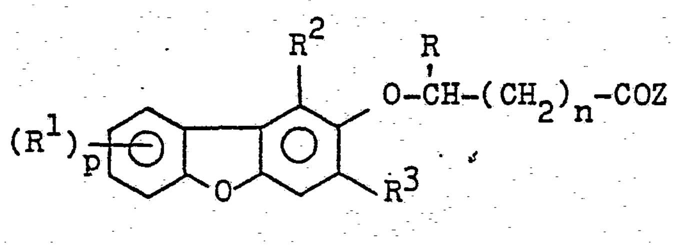

in welcher

Z ein Halogenatom bedeutet und $R^1$, $R^2$, $R^3$, R, n und p die in Anspruch 1 und 2 angegebenen Bedeutungen haben, gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers, bei Temperaturen von 0 bis 120°C umsetzt, oder

c) mit einem Dibenzofuranoxyalkancarbonsäureester der Formel

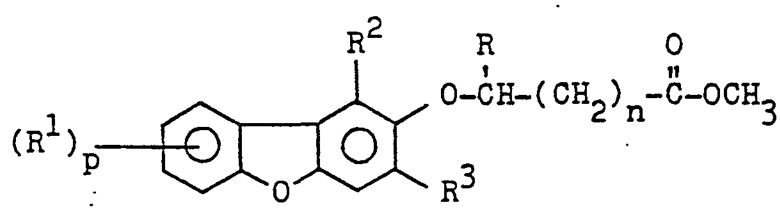

in welcher $R^1$, $R^2$, $R^3$, R, n und p die in Anspruch 1 angegebenen Bedeutungen haben, gegebenenfalls in Gegenwart eines Lösungs- oder Verdünnungsmittels, gegebenenfalls unter Zusatz einer starken anorganischen oder organischen Base oder einer starken anorganischen oder organischen Säure oder einer Lewis-Säure oder einem Metalloxid- oder -alkoholat bei Temperaturen von 0 bis 150°C umsetzt.

8. Verfahren zur Herstellung eines Dibenzofuranoxyalkancarbonsäureesters der allgemeinen Formel

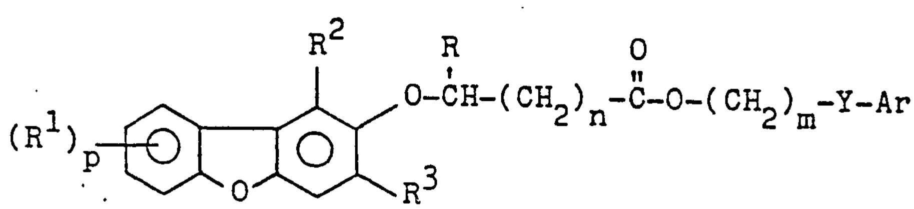

in welcher

$R^1$ Halogen, eine gegebenenfalls halogensubstituierte Alkyl- oder Alkoxygruppe mit 1 bis 4 C-Atomen, Cyan oder Nitro bedeutet,

$R^2$ und $R^3$ gleich oder verschieden sind und unabhängig voneinander Wasserstoff, Halogen oder Nitro bedeuten,

R Wasserstoff, Methyl oder Ethyl,

m die Zahlen 2, 3, 4, 5 oder 6

n die Zahlen 0 oder 2

p die Zahlen 1 oder 2 und

Y ein Sauerstoff- oder Schwefelatom bedeuten und

Ar 3-Dibenzofuranyl, 1,2- oder 1,4-Biphenyl, 1- oder 2-Naphthyl oder Phenyl bedeuten, wobei der Phenylrest durch Halogen, durch gegebenenfalls halogensubstituierte Alkyl-, Alkenyl-, Alkoxy- oder Alkylthiogruppen mit 1 bis 5 C-Atomen oder durch Acetyl, Cyan oder Nitro substituiert sein kann,

dadurch gekennzeichnet, daß man ein Dibenzofuranoxyalkancarbonsäure-Metallsalz der Formel

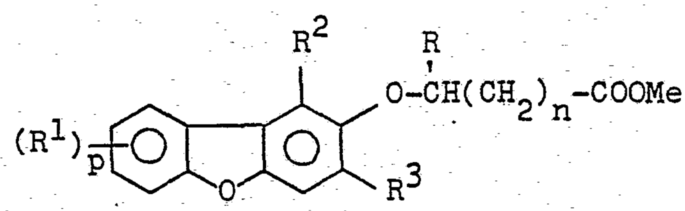

in welcher

$R^1$, $R^2$, $R^3$, R, Me, n und P die in den Ansprüchen 1 und 2 angegebenen Bedeutungen haben, mit einem Aryloxy- oder Arylthio-alkylhalogenid der Formel

$$Z-(CH_2)_m-Y-Ar$$

in welcher
Ar, Y, Z und m die in den Ansprüchen 1 und 2 angegebenen Bedeutungen haben, gegebenenfalls in Gegenwart eines Lösungs- oder Verdünnungsmittels und gegebenenfalls unter Zusatz eines Reaktionsbeschleunigers, bei Temperaturen zwischen 0 und 150°C umsetzt.

0035744

Europäisches Patentamt

# EUROPÄISCHER RECHERCHENBERICHT

Nummer der Anmeldung
EP 81 10 1546

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| D | DE - A - 2 732 924 (HOECHST)<br>* Patentansprüche *<br>--- | 1,3-6, 8 | C 07 D 307/91<br>A 01 N 43/12<br>//C 07 C 69/025 |
| D | DE - A - 2 823 424 (B.A.S.F.)<br>* Patentansprüche *<br>-------- | 1,3 | |

RECHERCHIERTE SACHGEBIETE (Int. Cl.³)

C 07 D 307/91
A 01 N 43/12
A 01 N 43/08

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | Juni 1981 | CHOULY |